(19) Europäisches Patentamt
European Patent Office
Office européen des brevets

(11) EP 1 082 610 B1

(12) # EUROPÄISCHE PATENTSCHRIFT

(45) Veröffentlichungstag und Bekanntmachung des Hinweises auf die Patenterteilung:
**19.09.2007 Patentblatt 2007/38**

(21) Anmeldenummer: **99920643.6**

(22) Anmeldetag: **12.04.1999**

(51) Int Cl.:
***G01N 33/28*** *(2006.01)* ***C10L 1/00*** *(2006.01)*

(86) Internationale Anmeldenummer:
**PCT/EP1999/002451**

(87) Internationale Veröffentlichungsnummer:
**WO 1999/056125 (04.11.1999 Gazette 1999/44)**

(54) **VERFAHREN ZUR DETEKTION VON MARKIERSTOFFEN IN KOHLENWASSERSTOFFEN UND KOHLENWASSERSTOFFGEMISCHEN**

Method for detecting marker substances in hydrocarbons and hydrocarbon mixtures

Procédé pour détecter des substances de marquage dans des hydrocarbures et des mélanges d'hydrocarbures

(84) Benannte Vertragsstaaten:
**AT BE CH DE DK ES FI FR GB GR IE IT LI NL PT SE**
Benannte Erstreckungsstaaten:
**LT LV RO SI**

(30) Priorität: **23.04.1998 DE 19818176**

(43) Veröffentlichungstag der Anmeldung:
**14.03.2001 Patentblatt 2001/11**

(73) Patentinhaber: **BASF Aktiengesellschaft**
**67056 Ludwigshafen (DE)**

(72) Erfinder:
- **MEYER, Frank**
  **D-69115 Heidelberg (DE)**
- **WAGENBLAST, Gerhard**
  **D-67157 Wachenheim (DE)**
- **BECK, Karin, Heidrun**
  **D-67067 Ludwigshafen (DE)**
- **VAMVAKARIS, Christos**
  **D-67169 Kallstadt (DE)**

(56) Entgegenhaltungen:
**US-A- 5 525 516**
**US-A- 5 698 397**
**US-A- 5 710 046**
**US-A- 5 723 338**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft ein Verfahren zur Detektion von Markierstoffen in Kohlenwasserstoffen und Kohlenwasserstoff gemischen welche mit mindestens zwei Markierstoffen markiert worden sind, wobei die Markierstoffe im Spektralbereich von 600 bis 1200 nm absorbieren und als Folge davon Fluoreszenzstrahlung emittieren und der Absorptionsbereich mindestens eines Markierstoffes mit dem Absorptionsbereich mindestens eines weiteren Markierstoffes überlappt.

Es ist häufig erforderlich, Flüssigkeiten zu markieren, um in der Folge, z.B. bei ihrer Anwendung, mittels geeigneter Methoden die so markierten Flüssigkeiten wieder zu detektieren. Beispielsweise kann auf diese Weise Heizöl, welches üblicherweise steuerlich begünstigt ist, von in der Regel höher versteuertem Dieselöl unterschieden werden oder es lassen sich flüssige Produktströme in großtechnischen Anlagen, wie z.B. Erdölraffinerien, markieren und dadurch verfolgen.

Soll die Markierung der Flüssigkeiten für das menschliche Auge unsichtbar sein, so ist man auf Markierstoffe angewiesen, welche außerhalb des sichtbaren Bereichs des Spektrums absorbieren und/oder Strahlung emittieren. Wegen der hohen Empfindlichkeit des Nachweises und damit verbunden der Möglichkeit, mit geringen Zugaben des Markierstoffs eine zuverlässige Markierung zu erreichen, sind hier vor allem Markierstoffe von Bedeutung, welche die absorbierte Strahlung als Fluoreszenzstrahlung wieder emittieren. In der Regel besitzt diese emittierte Strahlung dabei eine niedrigere Frequenz als die absorbierte Strahlung (STOKES-Strahlung), in selteneren Fällen dieselbe (Resonanz-Fluoreszenz) oder sogar eine höhere Frequenz (ANTI-STOKES-Strahlung).

Von großer (volks-)wirtschaftlicher Bedeutung ist die Markierung von Kohlenwasserstoffen und Kohlenwasserstoffgemischen (z.B. verschiedenste Qualitäten von Diesel- und Ottokraftstoffen sowie sonstigen Mineralölen). Da diese Flüssigkeiten üblicherweise im Spektralbereich unterhalb von etwa 600 nm selbst hohe Absorption und/oder Fluoreszenz aufweisen, werden sinnvollerweise Markierstoffe verwendet, welche oberhalb von etwa 600 nm absorbieren und/oder fluoreszieren.

Idealerweise besitzen daher Verbindungen, die als Markierstoffe verwendet werden sollen, folgende Grundvoraussetzungen:

Sie zeigen im Spektralbereich von 600 bis 1200 nm eine starke Absorption,

sie besitzen im sichtbaren Bereich des Spektrums dagegen keine oder nur geringfügige Absorption bzw. Fluoreszenz,

sie zeigen im Spektralbereich von etwa 600 bis etwa 1200 nm eine starke Emission von Fluoreszenzstrahlung,

die emittierte Fluoreszenzstrahlung läßt sich auch bei (gewichtsmäßigen) Konzentrationen der Markierstoffe in der betreffenden Flüssigkeit von weniger als 1 ppm noch zuverlässig detektieren und

sie besitzen in der zu markierenden Flüssigkeit eine ausreichende Löslichkeit.

Je nach spezieller Anforderung können für die Verbindungen, die als Markierstoffe verwendet werden sollen, noch ein oder mehrere der nachfolgend genannten Punkte von Bedeutung sein:

Sie sind mit anderen Markierstoffen und gegebenenfalls vorliegenden Additiven mischbar (dies gilt auch für die Mischbarkeit von markierten und gegebenenfalls additivierten Flüssigkeiten miteinander),

sie sind sowohl selbst als auch gelöst in der zu markierenden Flüssigkeit unter Einwirkung äußerer Bedingungen, wie z.B. Temperatur, Licht, Feuchtigkeit etc., stabil,

sie wirken nicht schädigend auf die Umgebung, wie z.B. Verbrennungsmotoren, Lagertanks etc., in welcher sie verwendet werden und

sie sind sowohl toxikologisch als auch ökologisch verträglich.

In der Schrift WO 94/02570 wird zur Markierung von Flüssigkeiten die Verwendung von Markierstoffen aus der Klasse der metallfreien oder metallhaltigen Phthalocyanine, der metallfreien oder metallhaltigen Naphthalocyanine, der Nickel-Dithiolen-Komplexe, der Aminiumverbindungen von aromatischen Aminen, der Methinfarbstoffe oder der Azulenquadratsäurefarbstoffe beschrieben, welche ihr Absorptionsmaximum im Bereich von 600 bis 1 200 nm und/oder ein Fluoreszenzmaximum im Bereich von 620 bis 1 200 nm aufweisen. Weiter wird ein Verfahren beschrieben, welches im

wesentlichen darin besteht, daß die Fluoreszenzstrahlung des in der Flüssigkeit vorhandenen Markierstoffs, welcher im besagten Spektralbereich Strahlung absorbiert, detektiert wird. Auch wird ein Detektor beschrieben, welcher zum Nachweis des Markierstoffs verwendet wird. Die Verwendung mehrerer Markierstoffe gleichzeitig wird jedoch nicht explizit erwähnt.

Die Schrift US 5,525,516 beschreibt ebenfalls ein Verfahren zur Markierung von Mineralölen mit Verbindungen, welche Fluoreszenzen im NIR aufweisen. Als solche Markierstoffe kommen substituierte Phthalocyanine, substituierte Naphthalocyanine sowie Quadrat-oder Krokonsäurederivate zur Verwendung. In der Beschreibung dieser US-Schrift (Spalte 3, Zeilen 35 bis 40) wird ausgeführt, daß im Rahmen der beschriebenen Erfindung ein oder mehrere Mineralöle nicht nur mit einer, sondern auch mit zweien oder mehreren im IR-Bereich fluoreszierenden Verbindungen markiert werden können. Weiter wird an dieser Stelle erwähnt, daß diese zwei oder mehr Verbindungen so ausgewählt werden, daß deren Wellenlängen der Absorption von IR-Strahlung und/oder Emission von Fluoreszenzstrahlung ausreichend weit auseinander liegen und sich bei der individuellen Detektion nicht gegenseitig beeinflussen. Als ausreichender Abstand der betreffenden Wellenlängen (Spalte 4, Zeilen 25 bis 28) werden dabei mit Detektionsgeräten entsprechend dem (damaligen) Stand der Technik etwa 20 nm angegeben. Eine Verwendung von Markierstoffen mit überlappenden Absorptionsbereichen wird in dieser Schrift nicht explizit offenbart. Es wird zudem darauf hingewiesen (Spalte 3, Zeilen 41 bis 44), daß diese fluoreszierende(n) Verbindung(en) vorzugsweise unterhalb von 850 nm absorbieren sollten, da oberhalb dieser Wellenlänge die Mineralöle Absorption zeigten.

Ferner wird in dieser Schrift ein Verfahren zur Identifizierung von Mineralölen beansprucht, welche mit einem oder mehreren Markierstoffen markiert worden sind. Hierbei wird ein Anregungsbereich (Absorptionsbereich) für das markierte Mineralöl bzw. die darin enthaltenen Markierstoffe von 670 bis 850 nm angegeben. Darüber hinaus werden keine weiteren Hinweise darauf geliefert, wie im Falle der Markierung von Mineralölen mit mehr als einem Markierstoff verfahren werden soll.

In der Schrift US 5,710,046 wird ein Verfahren zur Markierung von Benzin beschrieben, welches ebenfalls auf die Detektion eines im Benzin gelösten und im wesentlichen metallfreien Fluoreszenzfarbstoffs hinausläuft. Hierbei wird eine entsprechend markierte Benzinprobe mit Strahlung aus einem Wellenlängenband von 600 bis 2500 nm angeregt, das im Wellenlängenband von etwa 600 bis 2500 nm vom Farbstoff emittierte Fluoreszenzlicht detektiert und das resultierende Detektionssignal zur Identifizierung der markierten Probe herangezogen. Weiter wird in dieser Schrift ausführlich der Aufbau eines Detektors zum Nachweis der Fluoreszenzfarbstoffe in den markierten Benzinproben beschrieben. Auf die Verwendung von mehreren Markierstoffen (Farbstoffen) wird jedoch nicht eingegangen.

Sollen Flüssigkeiten, wie z.B. Kohlenwasserstoffe und Kohlenwasserstoffgemische (beispielsweise Diesel- und Ottokraftstoffe sowie sonstigen Mineralöle), unterschiedlicher Herkunft oder verschiedener Hersteller markiert werden, so benötigt man bei Verwendung von lediglich einem Markierstoff je Flüssigkeit eine Vielzahl verschiedener Markierstoffe. Diese müssen sich hinsichtlich ihres Absorptions- und/oder Fluoreszenzverhaltens dabei ausreichend voneinander unterscheiden, damit eine Identifizierung der Flüssigkeiten hinsichtlich ihrer Provenienz und/oder ihres Herstellers möglich wird. Durch die Markierung von Flüssigkeiten mit nur einem Markierstoff ist es zudem unbefugten Dritten leichter möglich, unmarkierte Flüssigkeiten durch Zugabe des entsprechenden Markierstoffs zu "fälschen". Dies ist von großer Bedeutung, wenn chemisch und qualitativ gleichwertige Flüssigkeiten mit unterschiedlichen Fiskalabgaben belegt sind. Beispielsweise seien hier nur Heizöl und Dieselkraftstoff genannt.

Aufgabe der vorliegenden Erfindung war es daher durch Zugabe von zwei oder mehr Markierstoffen Kohlenwasserstoffe und kohlenwasserstoffgemischem so zu markieren, d.h. so mit einem "Fingerabdruck" zu versehen, daß eine Nachstellung der Markierung erschwert wird.

Diese Aufgabe wurde gelöst durch ein Verfahren zur Markierung von Flüssigkeiten mit mindestens zwei Markierstoffen, welches dadurch gekennzeichnet ist, daß

die Markierstoffe im Spektralbereich von 600 bis 1200 nm absorbieren und als Folge davon Fluoreszenzstrahlung emittieren und

der Absorptionsbereich mindestens eines Markierstoffes mit dem Absorptionsbereich mindestens eines weiteren Markierstoffes überlappt.

Bevorzugt werden im erfindungsgemäßen Verfahren Markierstoffe verwendet, deren jeweilige Wellenlänge des Absorptionsmaximums im Spektralbereich von 600 bis 1200 nm liegt.

Durch die Verwendung von zwei oder mehr Markierstoffen, von welchen der Absorptionbereich mindestens eines Markierstoffs mit dem Absorptionsbereich mindestens eines weiteren Markierstoffes überlappt, ist es einerseits möglich, eine größere Anzahl von Markierstoffen im genannten Wellenlängenbereich einzusetzen. Weit wichtiger ist aber, daß die durch Dritte zur Nachahmung zu verwendenden ("falschen") Verbindungen nicht nur ähnliche Absorptionsmaxima wie die originalen Markierstoffe besitzen müssen, erstere müssen sich auch im übrigen Absorptionsbereich ähnlich wie letztere verhalten.

Beispielsweise nehme man an, daß jeder "falsche" Markierstoff jeweils nur ein, auf einen relativ engen Wellenlängenbereich beschränktes Absorptionsmaximum besitzt, welches dem eines originalen Markierstoffes entspricht. Weiter nehme man an, daß die originalen Markierstoffe darüber hinaus Absorptionsbereiche besitzen, welche zum Teil

überlappen. Verwendet man nun Strahlungsquellen, welche nur Strahlung in den Bereichen der Absorptionsmaxima emittieren, so sind in beiden Fällen ähnliche Fluoreszenzspektren zu erwarten. Werden jedoch Strahlungsquellen verwendet, welche bei Wellenlängen emittieren, bei welchen die "falschen" Markierstoffe keine Absorption zeigen, die originalen Markierstoffe aber überlappende Absorptionsbereiche aufweisen, so wird man in letzterem Fall, im Gegensatz zu ersterem Fall, von diesen Markierstoffen emittierte Fluoreszenzstrahlung erwarten.

In einem weiteren Beispiel sei angenommen, daß das Absorptionsmaximum eines originalen Markierstoffs M1 im überlappenden Absorptionsbereich aus demjenigen von M1 und demjenigen eines weiteren originalen Markierstoffs M2 liegt. Regt man nun die Markierstoffe M1 und M2 jeweils in ihren Absorptionsmaxima an, so resultiert das Fluoreszenzsignal von M1 nur aus dessen Anregung, das Fluoreszenzsignal des Markierstoffs M2 setzt sich jedoch zusammen aus dem Anteil von dessen individueller Anregung (im Absorptionsmaximum von M2) und einem weiteren Anteil, welcher aus der Anregung des Markierstoffs M1 (in dessen Absorptionsmaximum und gleichzeitig im überlappenden Absorptionsbereich der Markierstoffe M1 und M2) herrührt. Im Gegensatz dazu zeigen entsprechende "falsche" Markierstoffe, welche keine in solcher weise überlappenden Absorptionsbereiche aufweisen, bei Anregung in deren Absorptionsmaxima nur ihre jeweiligen, individuellen Fluoreszenzsignale. Ausführlicher wird hierauf weiter unten eingegangen.

Bevorzugt wird im Verfahren zur Markierung von Kohlenwasserstoffen und Kohlenwasserstoffgemischen eine Kombination von n Markierstoffen verwendet, wobei n eine ganze Zahl von 2 bis 10, also Werte von 2, 3, 4, 5, 6, 7, 8, 9 oder 10 bedeutet.

Besonders bevorzugt wird im Verfahren zur Markierung von Kohlenwasserstoffen und Kohlenwasserstoffgemischen eine Kombination von n Markierstoffen verwendet, wobei n eine ganze Zahl von 2 bis 6, also werte von 2, 3, 4, 5 oder 6 bedeutet.

Ganz besonders bevorzugt wird im Verfahren zur Markierung von Kohlenwasserstoffen und Kohlenwasserstoffgemischen eine Kombination von n Markierstoffen verwendet, wobei n eine ganze Zahl von 2 bis 4, also Werte von 2, 3 oder 4 bedeutet.

Als Markierstoffe setzt man im erfindungsgemäßen Verfahren sowie in den bevorzugten Ausführungsformen, in welchen man eine Kombination von n gleich 2 bis 10, n gleich 2 bis 6 oder n gleich 2 bis 4 Markierstoffen verwendet, vorzugsweise Verbindungen zu, die ausgewählt sind aus der Gruppe bestehend aus metallfreien und metallhaltigen Phthalocyaninen, metallfreien und metallhaltigen Naphthalocyaninen, Nickel-Dithiolen-Komplexen, Aminiumverbindungen von aromatischen Aminen, Methinfarbstoffen, Quadratsäurefarbstoffen und Krokonsäurefarbstoffen.

Geeignete Phthalocyanine gehorchen z.B. der Formel Ia

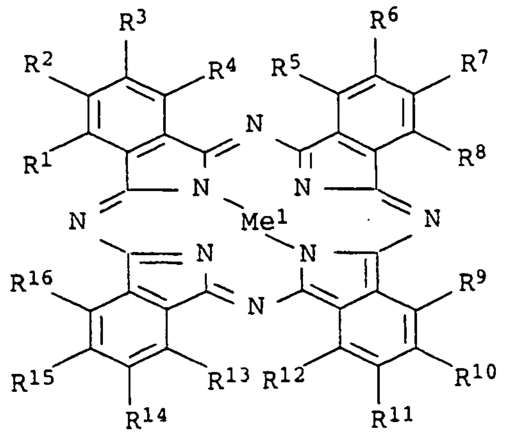

(Ia),

in der

$Me^1$ zweimal Wasserstoff, zweimal Lithium, Magnesium, Zink, Kupfer, Nickel, VO, TiO, AlCl, AlO-$C_1$-$C_{20}$-Alkyl, AlNH-$C_1$-$C_{20}$-Alkyl, AlN($C_1$-$C_{20}$-Alkyl)$_2$, AlO-$C_6$-$C_{20}$-Aryl, Al-NH-$C_6$-$C_{20}$-Aryl oder AlN(-$C_6$-$C_{20}$-Aryl)$_2$, AlN-Het, wobei N-Het ein heterocyclischer, gesättigter oder ungesättigter fünf-, sechs- oder siebengliedriger Ring ist, welcher neben mindestens einem Stickstoffatom noch ein oder zwei weitere Stickstoffatome und/oder ein weiteres Sauerstoff- oder Schwefelatom im Ring enthalten kann, welcher gegebenenfalls einbis dreifach mit $C_1$-$C_4$-Alkyl, Phenyl, Benzyl oder Phenylethyl substituiert ist und welcher über ein (bzw. das) Ringstickstoffatom an das Aluminiumatom gebunden ist, oder Si(OH)$_2$,

mindestens 4 der Reste $R^1$ bis $R^{16}$ unabhängig voneinander für einen Rest der Formel W-$X^1$, worin W für eine chemische Bindung, Sauerstoff, Schwefel, Imino, $C_1$-$C_4$-Alkylimino oder Phenylimino und $X^1$ für $C_1$-$C_{20}$-Alkyl oder

$C_3$-$C_{10}$-Cycloalkyl, das gegebenenfalls durch 1 bis 4 Sauerstoffatome in Etherfunktion unterbrochen ist und durch Phenyl substituiert sein kann, Adamantyl oder gegebenenfalls substituiertes Phenyl, heterocyclische, gesättigte fünf-, sechs-oder siebengliedrige Ringe, welche noch ein oder zwei weitere Stickstoffatome und/oder ein weiteres Sauerstoff- oder Schwefelatom im Ring enthalten können, welche gegebenenfalls ein- bis dreifach mit $C_1$-$C_4$-Alkyl, Phenyl, Benzyl oder Phenylethyl substituiert sind und welche über ein (bzw. das) Ringstickstoffatom an den Benzolring gebunden sind, stehen und

gegebenenfalls die übrigen Reste $R^1$ bis $R^{16}$ Wasserstoff, Halogen, Hydroxysulfonyl oder $C_1$-$C_4$-Dialkylsulfamoyl bedeuten.

Geeignete Phthalocyanine gehorchen weiterhin z.B. der Formel Ib

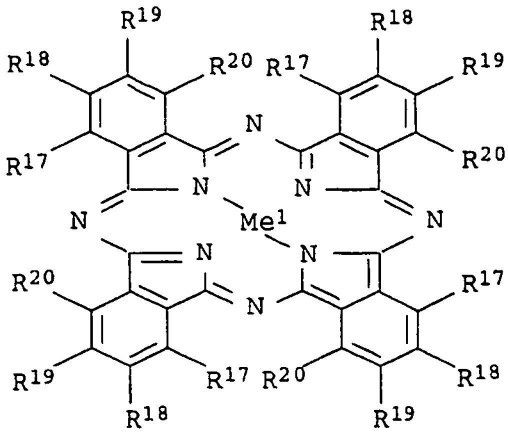

(Ib),

in der

$R^{17}$ und $R^{18}$ oder $R^{18}$ und $R^{19}$ oder $R^{19}$ und $R^{20}$ zusammen jeweils einen Rest der Formel $X^2$-$C_2H_4$-$X^3$, worin einer der beiden Reste $X^2$ und $X^3$ für Sauerstoff und der andere für Imino oder $C_1$-$C_4$-Alkylimino steht, und

$R^{19}$ und $R^{20}$ oder $R^{17}$ und $R^{20}$ oder $R^{17}$ und $R^{18}$ unabhängig voneinander jeweils Wasserstoff oder Halogen bedeuten und

$Me^1$ die obengenannte Bedeutung besitzt.

Weitere geeignete Phthalocyanine sind, sofern sie nicht bereits unter den oben aufgeführten Phthalocyaninen erwähnt wurden, in der Schrift US 5,526,516 unter der allgemeinen Formel I und exemplarisch in Tabelle 3 sowie in der Schrift US 5,703,229 unter der allgemeinen Formel II und exemplarisch in Tabelle 3 gezeigt.

Geeignete Naphthalocyanine gehorchen z.B. der Formel II

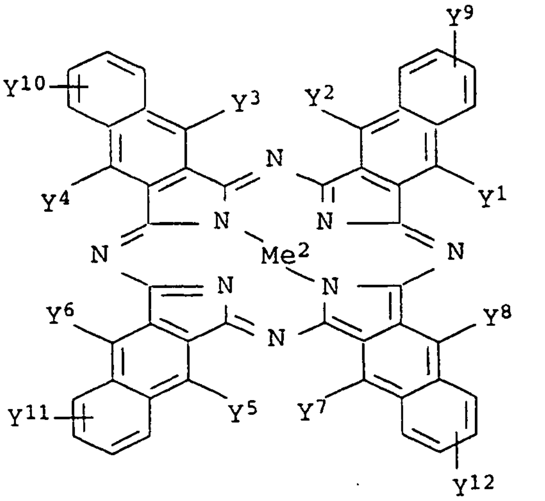

(II),

in der

$Y^1$, $Y^2$, $Y^3$, $Y^4$, $Y^5$, $Y^6$, $Y^7$ und $Y^8$ unabhängig voneinander jeweils wasserstoff, Hydroxy, $C_1$-$C_{20}$-Alkyl, $C_3$-$C_{10}$-Cycloalkyl oder $C_1$-$C_{20}$-Alkoxy, wobei die Alkylgruppen jeweils durch 1 bis 4 Sauerstoffatome in Etherfunktion unterbrochen sein können und gegebenenfalls durch Phenyl substituiert sind, heterocyclische, gesättigte fünf-, sechs- oder siebengliedrige Ringe, welche noch ein oder zwei weitere Stickstoffatome und/oder ein weiteres Sauerstoff- oder Schwefelatom im Ring enthalten können, welche gegebenenfalls ein- bis dreifach mit $C_1$-$C_4$-Alkyl, Phenyl, Benzyl oder Phenylethyl substituiert sind und welche über ein (bzw. das) Ringstickstoffatom an den Benzolring gebunden sind,

$Y^9$, $Y^{10}$, $Y^{11}$ und $Y^{12}$ unabhängig voneinander jeweils Wasserstoff, $C_1$-$C_{20}$-Alkyl oder $C_1$-$C_{20}$-Alkoxy, wobei die Alkylgruppen jeweils durch 1 bis 4 Sauerstoffatome in Etherfunktion unterbrochen sein können, Halogen, Hydroxysulfonyl oder $C_1$-$C_4$-Dialkylsulfamoyl und

$Me^2$ die Bedeutung von $Me^1$ besitzt oder den Rest

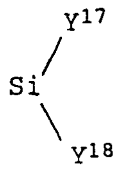

bedeutet, wobei

$Y^{17}$ und $Y^{18}$ unabhängig voneinander jeweils für Hydroxy, $C_1$-$C_{20}$-Alkoxy, $C_1$-$C_{20}$-Alkyl, $C_2$-$C_{20}$-Alkenyl, $C_3$-$C_{20}$-Alkenyloxy oder einen Rest der Formel

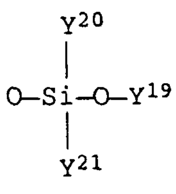

stehen, worin $Y^{19}$ die Bedeutung von $C_1$-$C_{20}$-Alkyl, $C_2$-$C_{20}$-Alkenyl oder $C_4$-$C_{20}$-Alkadienyl und $Y^{20}$ und $Y^{21}$ unab-

hängig voneinander jeweils die Bedeutung von $C_1$-$C_{12}$-Alkyl, $C_2$-$C_{12}$-Alkenyl oder des obengenannten Rests $OY^{19}$ besitzen.

Von besonderem Interesse sind dabei Naphthalocyanine der Formel II, in der mindestens einer der Reste $Y^1$ bis $Y^8$ von Wasserstoff verschieden sind.

weitere geeignete Naphthalocyanine sind, sofern sie nicht bereits unter den oben aufgeführten Naphthalocyaninen erwähnt wurden, in der Schrift US 5,526,516 unter der allgemeinen Formel II und exemplarisch in Tabelle 4 sowie in der Schrift US 5,703,229 unter der allgemeinen Formel III und exemplarisch in Tabelle 4 gezeigt.

Geeignete Nickel-Dithiolen-Komplexe gehorchen z.B. der Formel III

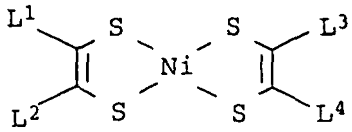

(III),

in der

$L^1$, $L^2$, $L^3$ und $L^4$ unabhängig voneinander jeweils $C_1$-$C_{20}$-Alkyl, das gegebenenfalls durch 1 bis 4 Sauerstoffatome in Etherfunktion unterbrochen ist, Phenyl, $C_1$-$C_{20}$-Alkylphenyl, $C_1$-$C_{20}$-Alkoxyphenyl, wobei die Alkylgruppen jeweils durch 1 bis 4 Sauerstoffatome in Etherfunktion unterbrochen sein können, oder $L^1$ und $L^2$ und/oder $L^3$ und $L^4$ jeweils zusammen den Rest der Formel

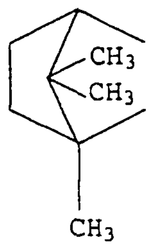

bedeuten.

Geeignete Aminiumverbindungen gehorchen z.B. der Formel IV

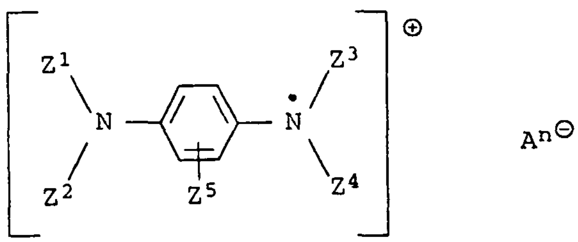

(IV),

in der

$Z^1$, $Z^2$, $Z^3$ und $Z^4$ unabhängig voneinander jeweils $C_1$-$C_{20}$-Alkyl, das gegebenenfalls durch 1 bis 4 Sauerstoffatome in Etherfunktion unterbrochen ist, $C_1$-$C_{20}$-Alkanoyl oder einen Rest der Formel

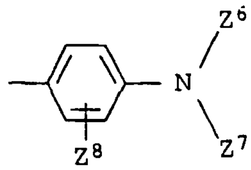

worin $Z^6$ für Wasserstoff, $C_1$-$C_{20}$-Alkyl, das gegebenenfalls durch 1 bis 4 Sauerstoffatome in Etherfunktion unterbrochen ist, oder $C_1$-$C_{20}$-Alkanoyl, $Z^7$ für Wasserstoff oder $C_1$-$C_{20}$-Alkyl, das gegebenenfalls durch 1 bis 4 Sauerstoffatome in Etherfunktion unterbrochen ist, und $Z^8$ für Wasserstoff, $C_1$-$C_{20}$-Alkyl, das gegebenenfalls durch 1 bis 4 Sauerstoffatome in Etherfunktion unterbrochen ist, oder Halogen stehen, und

$A^{n\ominus}$ das Äquivalent eines Anions bedeuten.

Geeignete Methinfarbstoffe gehorchen z.B. der Formel V

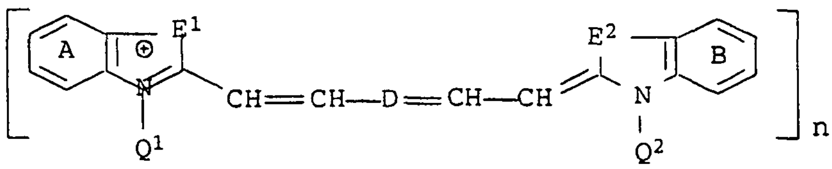

$A^{n\ominus}$ (V),

in der die Ringe A und B unabhängig voneinander jeweils gegebenenfalls benzoanelliert sind und substituiert sein können,

$E^1$ und $E^2$ unabhängig voneinander jeweils Sauerstoff, Schwefel, Imino oder einen Rest der Formel

$-C(CH_3)_2-$ oder $-CH=CH-$ ,

D einen Rest der Formel

$—CE^3=$ oder $—CH=CE^3—CH=$ , 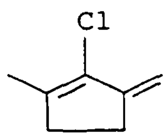 oder 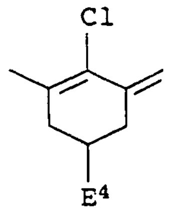 ,

worin $E^3$ für Wasserstoff, $C_1$-$C_6$-Alkyl, Chlor oder Brom und $E^4$ für Wasserstoff oder $C_1$-$C_6$-Alkyl stehen,

$Q^1$ und $Q^2$ unabhängig voneinander jeweils Phenyl, $C_5$-$C_7$-Cycloalkyl, $C_1$-$C_{12}$-Alkyl das durch 1 bis 3 Sauerstoffatome in Etherfunktion unterbrochen sein kann und gegebenenfalls durch Hydroxy, Chlor, Brom, Carboxyl, $C_1$-$C_4$-Alkoxycarbonyl, Acryloyloxy, Methacryloyloxy, Hydroxysulfonyl, $C_1$-$C_7$-Alkanoylamino, $C_1$-$C_6$-Alkylcarbamoyl, $C_1$-$C_6$-Alkylcarbamoyloxy oder einen Rest der Formel $G^{\oplus}(K)_3$, worin G für Stickstoff oder Phosphor und K für Phenyl, $C_5$-$C_7$-Cycloalkyl oder $C_1$-$C_{12}$-Alkyl stehen, substituiert sind,

$A^{n\ominus}$ das Äquivalent eines Anions und

n 1, 2 oder 3 bedeuten.

Geeignete Quadratsäurefarbstoffe sind z.B. solche Verbindungen, welche in der Schrift US 5,526,516 unter der allgemeinen Formel III gezeigt und exemplarisch in Tabelle 2 sowie in der Schrift US 5,703,229 unter der allgemeinen Formel IV und exemplarisch in Tabelle 2 aufgeführt sind.

Geeignete Quadratsäurefarbstoffe sind auch Azulenquadratsäurefarbstoffe, welche z.B. der nachstehend gezeigten Formel VI gehorchen

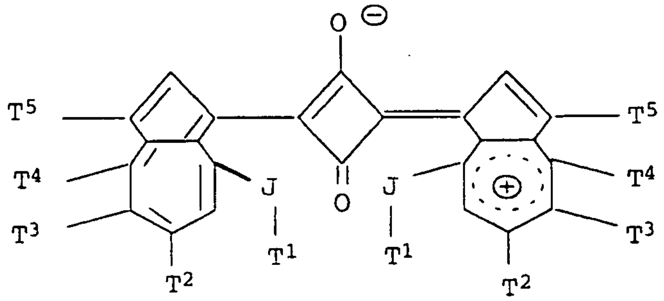

(VI),

in der

J $C_1$-$C_{12}$-Alkylen,

$T^1$ Wasserstoff, Halogen, Amino, Hydroxy, $C_1$-$C_{12}$-Alkoxy, Phenyl, substituiertes Phenyl, Carboxyl, $C_1$-$C_{12}$-Alkoxycarbonyl, Cyano oder einen Rest der Formel -$NT^7$-CO-$T^6$, -CO-$NT^6T^7$ oder O-CO-$NT^6T^7$, worin $T^6$ und $T^7$ unabhängig voneinander jeweils für Wasserstoff, $C_1$-$C_{12}$-Alkyl, $C_5$-$C_7$-Cycloalkyl, Phenyl, 2,2,6,6-Tetramethylpiperidin-4-yl oder Cyclohexylaminocarbonyl stehen, und

$T^2$, $T^3$, $T^4$ und $T^5$ unabhängig voneinander jeweils Wasserstoff oder $C_1$-$C_{12}$-Alkyl, das gegebenenfalls durch Halogen, Amino, $C_1$-$C_{12}$-Alkoxy, Phenyl, substituiertes Phenyl, Carboxyl, $C_1$-$C_{12}$-Alkoxycarbonyl oder Cyano substituiert ist, bedeuten,

mit der Maßgabe, daß wenn $T^5$ Wasserstoff bedeutet, an einem oder beiden Azulenringen die Ringpositionen der Substituenten J-$T^1$ und $T^4$ innerhalb eines Azulenrings auch gegeneinander vertauscht sein können.

Geeignete Quadratsäurefarbstoffe sind z.B. auch solche Verbindungen, welche der nachstehenden Formel VIa gehorchen,

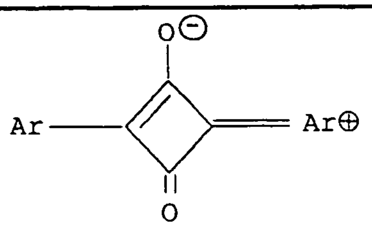

(VIa),

in der Ar jeweils unabhängig voneinander für einen gegebenenfalls mit $C_1$-$C_{20}$-Alkoxy, $C_1$-$C_{20}$-Alkylamino, $C_1$-$C_{20}$-Dialkylamino oder $C_1$-$C_{20}$-Alkylthio substituierten, aromatischen oder heteroaromatischen fünf- oder sechsgliedrigen Ring, wie z.B. Phenyl, Naphthyl, Thiophen, Pyridin oder Thiazol steht. Die Alkylgruppen können jeweils durch 1 bis 4 Sauerstoffatome in Etherfunktion unterbrochen und gegebenenfalls durch Phenyl substituiert sein.

Ar ist bevorzugt Phenyl, welches in 2-, 2,4- oder 2,4,6-Stellung mit den besagten Resten mono-, di- bzw. trisubstituiert ist. Vorzugsweise sind bei mehrfacher Sustitution des Phenyls diese Reste gleich. Insbesondere kommen in Betracht solche Verbindungen, in welchen beide Ar gleich sind.

Geeignete Krokonsäurefarbstoffe sind z.B. solche Verbindungen, welche in der Schrift US 5,526,516 unter der allgemeinen Formel IV gezeigt und exemplarisch in Tabelle 5 aufgeführt sind.

Alle in den obengenannten Formeln auftretenden Alkyl-, Alkylen-oder Alkenylreste können sowohl geradkettig als auch verzweigt sein.

In Formel Ia, II, III, IV oder VIa sind geeignete $C_1$-$C_{20}$-Alkylreste, die gegebenenfalls durch 1 bis 4 Sauerstoffatome in Etherfunktion unterbrochen sind, z.B. Methyl, Ethyl, Propyl, Isopropyl, Butyl, Isobutyl, sec-Butyl, tert-Butyl,

Pentyl, Isopentyl, Neopentyl, tert-Pentyl, Hexyl, 2-Methylpentyl, Heptyl, Octyl, 2-Ethylhexyl, Isooctyl, Nonyl, Isononyl, Decyl, Isodecyl, Undecyl, Dodecyl, Tridecyl, 3,5,5,7-Tetramethylnonyl, Isotridecyl (die obigen Bezeichnungen Isooctyl, Isononyl, Isodecyl und Isotridecyl sind Trivialbezeichnungen und stammen von den nach der Oxosynthese erhaltenen Alkoholen - vgl. dazu Ullmanns Encyklopädie der technischen Chemie, 4. Auflage, Band 7, Seiten 215 bis 217, sowie Band 11, Seiten 435 und 436), Tetradecyl, Pentadecyl, Hexadecyl, Heptadecyl, Octadecyl, Nonadecyl, Eicosyl, 2-Methoxyethyl, 2-Ethoxyethyl, 2-Propoxyethyl, 2-Isopropoxyethyl, 2-Butoxyethyl, 2- oder 3-Methoxypropyl, 2- oder 3-Ethoxypropyl, 2- oder 3-Propoxypropyl, 2- oder 3-Butoxypropyl, 2- oder 4-Methoxybutyl, 2- oder 4-Ethoxybutyl, 2- oder 4-Propoxybutyl, 2- oder 4-Butoxybutyl, 3,6-Dioxaheptyl, 3,6-Dioxaoctyl, 4,8-Dioxanonyl, 3,7-Dioxaoctyl, 3,7-Dioxanonyl, 4,7-Dioxaoctyl, 4,7-Dioxanonyl, 4,8-Dioxadecyl, 3,6,8-Trioxadecyl, 3,6,9-Trioxaundecyl, 3,6,9,12-Tetraoxatridecyl oder 3,6,9,12-Tetraoxatetradecyl.

In Formel Ia oder II sind geeignete $C_3$-$C_{10}$-Cycloalkylreste verzweigte oder unverzweigte Cycloalkylreste, die gegebenenfalls durch 1 bis 4 Sauerstoffatome in Etherfunktion unterbrochen sind, z.B. Cyclopropyl, Cyclobutyl, Cyclopentyl, Tetrahydrofuranyl, Cyclohexyl, Tetrahydropyranyl, Cycloheptyl, Oxepanyl, 1-Methylcyclopropyl, 1-Ethylcyclopropyl, 1-Propylcyclopropyl, 1-Butylcyclopropyl, 1-Pentylcyclopropyl, 1-Methyl-1-Butylcyclopropyl, 1,2-Dimethylcyclypropyl, 1-Methyl-2-Ethylcyclopropyl, Cyclooctyl, Cyclononyl oder Cyclodecyl.

In Formel Ia, Ib oder II sind geeignete $C_6$-$C_{20}$-Arylreste in den $A_{10}$-$C_6$-$C_{20}$-Aryl-, $A_1$-NH-$C_6$-$C_{20}$-Aryl- oder AlN (-$C_6$-$C_{20}$-Aryl)$_2$-Gruppierungen von $Me^1$ bzw. $Me^2$ z.B. gegebenenfalls mit bis zu zwei $C_1$-$C_7$-Alkyl-, mit bis zu drei $C_1$-$C_4$-Alkyl-, mit bis zu vier $C_1$-$C_3$-Alkyl- oder mit bis zu fünf Methyl- oder Ethylresten substituiertes Phenyl oder gegebenenfalls mit bis zu zwei $C_1$-$C_5$-Alkyl, mit bis zu drei $C_1$-$C_3$-Alkyl- oder mit bis zu vier Methyl- oder Ethylresten substituiertes Naphthyl, wobei solche gegebenenfalls vorhandenen Alkylsubstituenten bereits unter den vorher aufgeführten $C_1$-$C_{20}$-Alkylresten genannt sind.

In Formel Ia, Ib oder II leiten sich geeignete N-Het in den AlN-Het-Gruppierungen von $Me^1$ bzw. $Me^2$ ab von z.B. Pyrrol, Pyrrolidin, Pyrazol, Pyrazolidin, Imidazol, Imidazolin, 1H-1, 2, 3-Triazol, 1,2,3-Triazolidin, 1H-1,2,4-Triazol, 1,2,4-Triazolidin, Pyridin, Piperidin, Pyrazin, Piperazin, Pyridazin, Morpholin, 1H-Azepin, 2H-Azepin, Azepan, Oxazol, Oxazolidin, Thiazol, Thiazolidin, 1,2,3-, 1,2,4- oder 1,3,4-Oxadiazol, 1,2,3-, 1,2,4- oder 1, 3, 4-Oxadiazolidin, 1,2,3-, 1,2,4- oder 1,3,4-Thiadiazol oder 1,2,3-, 1,2,4- oder 1,3,4-Thiadiazolidin, wobei die heterocyclischen Ringe gegebenenfalls ein- bis dreifach mit $C_1$-$C_4$-Alkyl, Phenyl, Benzyl oder Phenylethyl substituiert sind. Entsprechende, gegebenenfalls in Frage kommende $C_1$-$C_4$-Alkylreste wurden bereits oben bei den $C_1$-$C_{20}$-Alkylresten genannt.

In Formel Ia oder II leiten sich geeignete heterocyclische, ringförmige Reste für $R^1$ bis $R^{16}$ bzw. $Y^1$ bis $Y^8$ ab von heterocyclischen, gesättigten fünf-, sechs- oder siebengliedrigen Ringen, welche noch ein oder zwei weitere Stickstoffatome und/oder ein weiteres Sauerstoff- oder Schwefelatom im Ring enthalten können, z.B. Pyrrolidin, Pyrazolidin, Imidazolin, 1,2,3-Triazolidin, 1,2,4-Triazolidin, Piperidin, Piperazin, Morpholin, Azepan, Oxazolidin, Thiazolidin, 1,2,3-, 1,2,4- oder 1,3,4-Oxadiazolidin, oder 1,2,3-, 1,2,4- oder 1,3,4-Thiadiazolidin, wobei die heterocyclischen Ringe gegebenenfalls ein- bis dreifach mit $C_1$-$C_4$-Alkyl, Phenyl, Benzyl oder Phenylethyl substituiert sind. Entsprechende, gegebenenfalls in Frage kommende $C_1$-$C_4$-Alkylreste wurden bereits oben bei den $C_1$-$C_{20}$-Alkylresten genannt.

In Formel Ia, II oder VIa ist geeignetes $C_1$-$C_{20}$-Alkyl, das durch Phenyl substituiert ist, z.B. Benzyl oder 1- oder 2-Phenylethyl.

In Formel II, III, IV oder VIa sind geeignete $C_1$-$C_{20}$-Alkoxyreste, die gegebenenfalls durch 1 bis 4 Sauerstoffatome in Etherfunktion unterbrochen sind, z.B. Methoxy, Ethoxy, Propoxy, Isopropoxy, Butoxy, Isobutoxy, Pentyloxy, Hexyloxy, Heptyloxy, Octyloxy, 2-Ethylhexyloxy, Isooctyloxy, Nonyloxy, Isononyloxy, Decyloxy, Isodecyloxy, Undecyloxy, Dodecyloxy, Tridecyloxy, Isotridecyloxy, Tetradecyloxy, Pentadecyloxy, Hexadecyloxy, Heptadecyloxy, Octadecyloxy, Nonadecyloxy, Eicosyloxy, 2-Methoxyethoxy, 2-Ethoxyethoxy, 2-Propoxyethoxy, 2-Isopropoxyethoxy, 2-Butoxyethoxy, 2-oder 3-Methoxypropoxy, 2- oder 3-Ethoxypropoxy, 2- oder 3-Propoxypropoxy, 2- oder 3-Butoxypropoxy, 2-oder 4-Methoxybutoxy, 2-oder 4-Ethoxybutoxy, 2- oder 4-Propoxybutoxy, 2- oder 4-Butoxybutoxy, 3,6-Dioxaheptyloxy, 3,6-Dioxaoctyloxy, 4,8-Dioxanonyloxy, 3,7-Dioxaoctyloxy, 3,7-Dioxanonyloxy, 4,7-Dioxaoctyloxy, 4,7-Dioxanonyloxy, 4,8-Dioxadecyloxy, 3,6,8-Trioxadecyloxy, 3,6,9-Trioxaundecyloxy, 3,6,9,12-Tetraoxatridecyloxy oder 3,6,9,12-Tetraoxatetradecyloxy.

In Formel II oder VIa ist geeignetes $C_1$-$C_{20}$-Alkoxy, das durch Phenyl substituiert ist, z.B. Benzyloxy oder 1-oder 2-Phenylethoxy.

In Formel Ia, III oder VI ist geeignetes substituiertes Phenyl z.B. durch $C_1$-$C_6$-Alkyl, $C_1$-$C_6$-Alkoxy, Hydroxy oder Halogen substituiertes Phenyl. In der Regel können dabei 1 bis 3 Substituenten auftreten. Insbesondere ist das Phenyl mit 1 oder mit 2 Substituenten $C_1$-$C_6$-Alkyl oder $C_1$-$C_6$-Alkoxy substituiert. Bei Monosubstitution befindet sich der Substituent vorzugsweise in paraStellung. Bei Disubstitution sind die Substituenten vorzugsweise in 2,3-, 2,4-, 3,4- und 3,5-Stellung.

Halogen in Formel Ib, II, IV oder VI ist z.B. Fluor, Chlor oder Brom.

Reste W in Formel Ia sowie $X^2$ oder $X^3$ in Formel Ib sind z.B. Methylimino, Ethylimino, Propylimino, Isopropylimino oder Butylimino.

Reste $R^1$ bis $R^{16}$ in Formel Ia sowie $Y^9$ bis $Y^{12}$ in Formel II sind z.B. Dimethylsulfamoyl, Diethylsulfamoyl,

Dipropylsulfamoyl, Dibutylsulfamoyl oder N-Methyl-N-ethylsulfamoyl.

$C_2$-$C_{20}$-Alkenyl sowie $C_4$-$C_{20}$-Alkandienyl in Formel II ist z.B. Vinyl, Allyl, Prop-1-en-1-yl, Methallyl, Ethallyl, But-3-en-1-yl, Pentenyl, Pentadienyl, Hexadienyl, 3,7-Dimethylocta-1,6-dien-1-yl, Undec-10-en-1-yl, 6,10-Dimethylundeca-5,9-dien-2-yl, Octadec-9-en-1-yl, Octadeca-9,12- dien-1-yl, 3,7,11,15-Tetrame-thylhexadec-1-en-3-yl oder Eicos-9-en-1-yl.

$C_3$-$C_{20}$-Alkenyloxy in Formel II ist z.B. Allyloxy, Methallyloxy, But-3-en-1-yloxy, Undec-10-en-1-yloxy, Octadec-9-en-1-yloxy oder Eicos-9-en-1-yloxy.

$Z^6$ in Formel IV bedeutet z.B. Formyl, Acetyl, Propionyl, Butyryl, Isobutyryl, Pentanoyl, Hexanoyl, Heptanoyl, Octanoyl oder 2-Ethylhexanoyl.

Wenn die Ringe A und/oder B in Formel V substituiert sind, so können als Substituenten z.B. $C_1$-$C_6$-Alkyl, Phenyl-$C_1$-$C_6$-alkoxy, Phenoxy, Halogen, Hydroxy, Amino, $C_1$-$C_6$-Mono- oder Dialkylamino oder Cyano in Betracht kommen. Die Ringe sind dabei in der Regel 1 bis 3-fach substituiert.

Reste $E^3$, $E^4$, $Q^1$ und $Q^2$ in Formel V sind z.B. Methyl, Ethyl, Propyl, Isopropyl, Butyl, Isobutyl, sec-Butyl, Pentyl, Isopentyl, Neopentyl, tert-Pentyl oder Hexyl.

Reste $Q^1$ und $Q^2$ sind weiterhin z.B. Hexyl, 2-Methylpentyl, Heptyl, Octyl, 2-Ethylhexyl, Isooctyl, Nonyl, Isononyl, Decyl, Isodecyl, Undecyl, Dodecyl, Cyclopentyl, Cyclohexyl, 2-Methoxyethyl, 2-Ethoxyethyl, 2- oder 3-Methoxypropyl, 2- oder 3-Ethoxypropyl, 2-Hydroxyethyl, 2- oder 3-Hydroxypropyl, 2-Chlorethyl, 2-Bromethyl, 2- oder 3-Chlorpropyl, 2- oder 3-Brompropyl, 2-Carboxyethyl, 2- oder 3-Carboxypropyl, 2-Methoxycarbonylethyl, 2-Ethoxycarbonylethyl, 2- oder 3-Methoxycarbonylpropyl, 2- oder 3-Ethoxycarbonylpropyl, 2-Acryloyloxyethyl, 2- oder 3-Acryloyloxypropyl, 2-Methacryloyloxyethyl, 2- oder 3-Methacryloyloxypropyl, 2-Hydroxysulfonylethyl, 2- oder 3-Hydroxysulfonylpropyl, 2-Acetylaminoethyl, 2- oder 3-Acetylaminopropyl, 2-Methylcarbamoylethyl, 2-Ethylcarbamoylethyl, 2- oder 3-Methylcarbamoylpropyl, 2- oder 3-Ethylcarbamoylpropyl, 2-Methylcarbamoyloxyethyl, 2-Ethylcarbamoyloxyethyl, 2- oder 3-Methylcarbamoyloxy- propyl, 2- oder 3-Ethylcarbamoyloxypropyl, 2-(Trimethylammonium)ethyl, 2-(Triethylammonium)ethyl, 2- oder 3-(Trimethylammonium)propyl, 2- oder 3-(Triethylammonium)propyl, 2-(Triphenylphosphonium)ethyl oder 2-oder 3-(Triphenylphosphonium)propyl.

$A^{n\ominus}$ in Formel IV oder V leitet sich z.B. von Anionen organischer oder anorganischer Säuren ab. Besonders bevorzugt sind dabei z.B. Methansulfonat, 4-Methylbenzolsulfonat, Acetat, Trifluoroacetat, Heptafluorobutyrat, Chlorid, Bromid, Iodid, Perchlorat, Tetrafluoroborat, Nitrat, Hexafluorophosphat oder Tetraphenylborat.

Reste J in Formel VI sind z.B. Methylen, Ethylen, 1,2- oder 1,3-Propylen, 1,2-, 1,3-, 2,3- oder 1,4-Butylen, Pentamethylen, Hexamethylen, Heptamethylen, Octamethylen, Nonamethylen, Decamethylen, Undecamethylen oder Dodecamethylen.

Reste $T^2$, $T^3$, $T^4$ und $T^5$ in Formel VI sind beispielsweise Methyl, Ethyl, Propyl, Isopropyl, Butyl, Isobutyl, sec-Butyl, tert-Butyl, Pentyl, Isopentyl, Neopentyl, tert-Pentyl, 2-Methylbutyl, Hexyl, 2-Methylpentyl, Heptyl, Octyl, 2-Ethylhexyl, Isooctyl, Nonyl, Isononyl, Decyl, Undecyl, Dodecyl, Fluormethyl, Chlormethyl, Difluormethyl, Trifluormethyl, Trichlormethyl, 2-Fluorethyl, 2-Chlorethyl, 2-Bromethyl, 1,1,1-Trifluorethyl, Heptafluorpropyl, 4-Chlorbutyl, 5-Fluorpentyl, 6-Chlorhexyl, Cyanomethyl, 2-Cyanoethyl, 3-Cyanopropyl, 2-Cyanobutyl, 4-Cyanobutyl, 5-Cyanopentyl, 6-Cyanohexyl, 2-Aminoethyl, 2-Aminopropyl, 3-Aminopropyl, 2-Aminobutyl, 4-Aminobutyl, 5-Aminopentyl, 6-Aminohexyl, 2-Hydroxyethyl, 2-Hydroxypropyl, 3-Hydroxypropyl, 2-Hydroxybutyl, 4-Hydroxybutyl, 5-Hydroxypentyl, 6-Hydroxyhexyl, 2-Methoxyethyl, 2-Ethoxyethyl, 2-Propoxyethyl, 2-Isopropoxyethyl, 2-Butoxyethyl, 2-Methoxypropyl, 2-Ethoxypropyl, 3-Ethoxypropyl, 4-Ethoxybutyl, 4-Isopropoxybutyl, 5-Ethoxypentyl, 6-Methoxyhexyl, Benzyl, 1-Phenylethyl, 2-Phenylethyl, 4-Chlorbenzyl, 4-Methoxybenzyl, 2-(4-Methylphenyl)ethyl, Carboxymethyl, 2-Carboxyethyl, 3-Carboxypropyl, 4-Carboxybutyl, 5-Carboxypentyl, 6-Carboxyhexyl, Methoxycarbonylmethyl, Ethoxycarbonylmethyl, 2-Methoxycarbonylethyl, 2-Ethoxycarbonylethyl, 3-Methoxycarbonylpropyl, 3-Ethoxycarbonylpropyl, 4-Methoxycarbonylbutyl, 4-Ethoxycarbonylbutyl, 5-Methoxycarbonylpentyl, 5-Ethoxycarbonylpentyl, 6-Methoxycarbonylhexyl oder 6-Ethoxycarbonylhexyl.

$T^1$ in Formel VI ist z.B. Methoxycarbonyl, Ethoxycarbonyl, Propoxycarbonyl, Isopropoxycarbonyl, Butoxycarbonyl, Isobutoxycarbonyl, sec-Butoxycarbonyl, tert-Butoxycarbonyl, Pentyloxycarbonyl, Isopentyloxycarbonyl, Neopentyloxycarbonyl, tert-Pentyloxycarbonyl, Hexyloxycarbonyl, Heptyloxycarbonyl, Octyloxycarbonyl, Isooctyloxycarbonyl, Nonyloxycarbonyl, Isononyloxycarbonyl, Decyloxycarbonyl, Isodecyloxycarbonyl, Undecyloxycarbonyl, Dodecyloxycarbonyl, Methoxy, Ethoxy, Propoxy, Isopropoxy, Butoxy, Isobutoxy, Pentyloxy, Hexyloxy, Acetylamino, Carbamoyl, Mono- oder Dimethylcarbamoyl, Mono- oder Diethylcarbamoyl, Monocyclohexylcarbonyl, Phenylcarbamoyl, Dimethylcarbamoyloxy oder Diethylcarbamoyloxy.

Als Markierstoffe sind weiter besonders hervorzuheben die Naphthalocyanine der Formel IIa

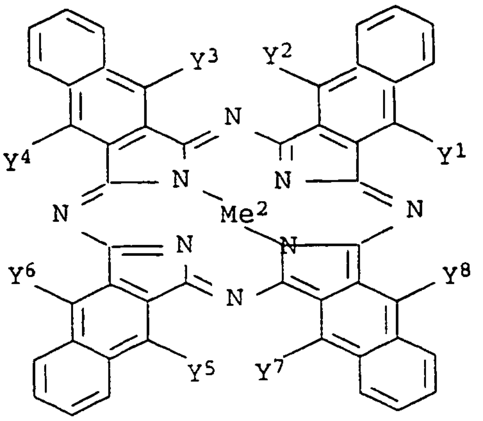

(IIa),

in der

$Y^1$, $Y^2$, $Y^3$, $Y^4$, $Y^5$, $Y^6$, $Y^7$ und $Y^8$ unabhängig voneinander jeweils Wasserstoff, Hydroxy, $C_1$-$C_4$-Alkyl oder $C_1$-$C_{20}$-Alkoxy und

$Me^2$ die Bedeutung von $Me^1$ hat oder dem Rest

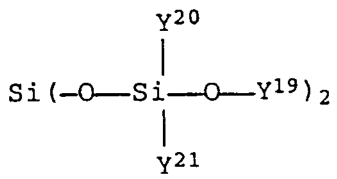

entspricht, worin $R^{19}$ für $C_1$-$C_{13}$-Alkyl oder $C_{10}$-$C_{20}$-Alkadienyl und $Y^{20}$ und $Y^{21}$ unabhängig voneinander jeweils für $C_1$-$C_{13}$-Alkyl oder $C_2$-$C_4$-Alkenyl stehen.

Besonders hervorzuheben sind hierbei Naphthalocyanine der Formel IIa, in der $Y^1$, $Y^2$, $Y^3$, $Y^4$, $Y^5$, $Y^6$, $Y^7$ und $Y^8$ unabhängig voneinander jeweils Hydroxy, $C_1$-$C_{20}$-Alkoxy, insbesondere $C_1$-$C_{10}$-Alkoxy bedeuten. Die Alkoxyreste können dabei gleich oder verschieden sein. Weiter sind besonders hervorzuheben Naphthalocyanine der Formel IIa, in der $Me^2$ zweimal Wasserstoff bedeutet.

Als Markierstoffe sind weiter hervorzuheben Nickel-Dithiolen-Komplexe der Formel III, in der $L^1$, $L^2$, $L^3$ und $L^4$ unabhängig voneinander jeweils Phenyl, $C_1$-$C_{20}$-Alkylphenyl, $C_1$-$C_{20}$-Alkoxyphenyl oder durch Hydroxy und $C_1$-$C_{20}$-Alkyl substituiertes Phenyl oder $L^1$ und $L^2$ sowie $L^3$ und $L^4$ jeweils zusammen den Rest der Formel

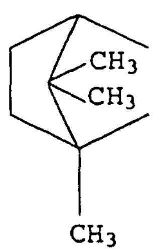

bedeuten.

Besonders hervorzuheben sind hierbei Nickel-Dithiolen-Komplexe der Formel III, in der $L^1$ und $L^4$ jeweils Phenyl und $L^2$ und $L^4$ jeweils einen Rest der Formel 4-$[C_2H_5$-$C(CH_3)_2]$-$C_6H_4$ bedeuten.

Bevorzugt werden im erfindungsgemäßen Verfahren und in den bevorzugten Ausführungsformen als Markierstoffe die oben gezeigten Phthalocyanine der Formel Ia sowie die in der Schrift US 5,525,516 in Tabelle 3 aufgeführten Phthalocyanine, die oben gezeigten Naphthalocyanine der Formel II, die in der Schrift US 5,525,516 in Tabelle 4 aufgeführten Naphthalocyanine und besonders bevorzugt die oben gezeigten Naphthalocyanine der Formel IIa verwendet. Besonders hervorzuheben sind hierbei Phthalocyanine und Naphthalocyanine, in welchen $Me^1$ bzw. $Me^2$ zweimal Wasserstoff bedeutet.

Im Spektralbereich von 600 bis etwa 850 nm verwendet man dabei üblicherweise Phthalocyanine, im Spektralbereich oberhalb von 5 etwa 800 nm üblicherweise Naphthalocyanine.

Die Phthalocyanine der Formel Ia sind an sich bekannt und z.B. in DE-B-1 073 739 oder EP-A-155 780 beschrieben oder können nach an sich bekannten Methoden, wie sie bei der Herstellung von Phthalocyaninen oder Naphthalocyaninen zur Anwendung kommen und wie sie beispielsweise in F.H. Moser, A.L. Thomas "The Phthalocyanines", CRC Press, Boca Rota, Florida, 1983, oder J. Am. Chem. Soc. Band 106, Seiten 7404 bis 7410, 1984, beschrieben sind, erhalten werden. Die Phthalocyanine der Formel Ib sind ebenfalls an sich bekannt und z.B. in EP-A-155 780 beschrieben oder können gemäß den Methoden des obengenannten Standes der Technik (Moser, J.Am. Chem.Soc.) erhalten werden.

Die Naphthalocyanine der Formel II sind ebenfalls an sich bekannt und beispielsweise in der EP-A-336 213, EP-A-358 080, GB-A-2 168 372 oder GB-A-2 200 650 beschrieben oder können gemäß den Methoden des obengenannten Standes der Technik (Moser, J.Am. Chem.Soc.) erhalten werden.

Die Nickel-Dithiolen-Komplexe der Formel III sind ebenfalls an sich bekannt und beispielsweise in der EP-A-192 215 beschrieben.

Die Aminiumverbindungen der Formel IV sind ebenfalls an sich bekannt und z.B. in US-A-3 484 467 beschrieben oder können gemäß den dort genannten Methoden erhalten werden.

Die Methinfarbstoffe der Formel V sind ebenfalls an sich bekannt und z.B. in der EP-A-464 543 beschrieben oder können gemäß den dort genannten Methoden erhalten werden.

Die Herstellung der Quadratsäurefarbstoffe ist in den Schriften US 5,525,516 und US 5,703,229 und der jeweils darin zitierten Literatur beschrieben.

Die Herstellung der Krokonsäurefarbstoffe ist in der Schrift US 5,525,516 und der darin zitierten Literatur beschrieben.

Die Azulenquadratsäurefarbstoffe der Formel VI sind ebenfalls an sich bekannt und z.B. in der EP-A-310 080 oder US-A-4 990 649 beschrieben oder können gemäß den dort genannten Methoden erhalten werden.

Flüssigkeiten, die man nach dem Verfahren mit einer Kombination aus mindestens zwei der oben näher bezeichneten Verbindungen als Markierstoffe markieren kann, sind üblicherweise 5 organische Flüssigkeiten, beispielsweise Alkohole, wie Methanol, Ethanol, Propanol, Isopropanol, Butanol, Isobutanol, sec-Butanol, Pentanol, Isopentanol, Neopentanol oder Hexanol,

Glykole, wie 1,2-Ethylenglykol, 1,2- oder 1,3-Propylenglykol, 1,2-, 2,3- oder 1,4-Butylenglykol, Di- oder Triethylenglykol oder Di- oder Tripropylenglykol,

Ether, wie Methyl-tertbutylether, 1,2-Ethylenglykolmono- oder -dimethylether, 1,2-Ethylenglykolmono- oder -diethylether, 3-Methoxypropanol, 3-Isopropoxypropanol, Tetrahydrofuran oder Dioxan,

Ketone, wie Aceton, Methylethylketon oder Diacetonalkohol,

Ester, wie Essigsäuremethylester, Essigsäureethylester, Essigsäurepropylester oder Essigsäurebutylester,

aliphatische oder aromatische Kohlenwasserstoffe wie Pentan, Hexan, Heptan, Octan, Isooctan, Petrolether, Toluol, Xylol, Ethylbenzol, Tetralin, Dekalin, Dimethylnaphthalin, Testbenzin,

natürliche Öle, wie Olivenöl, Sojaöl oder Sonnenblumenöl, oder natürliche oder synthetische Motoren-, Hydraulik- oder Getriebeöle, z.B. Fahrzeugmotorenöl oder Nähmaschinenöl, oder Bremsflüssigkeiten

und Mineralöle, wie Benzin, Kerosin, Dieselöl oder Heizöl.

Besonders vorteilhaft verwendet man die obengenannten Verbindungen zum Markieren von Mineralölen, bei denen gleichzeitig eine Kennzeichnung gefordert wird, z.B. aus steuerlichen Gründen. Um die Kosten der Kennzeichnung gering zu halten aber auch um mögliche Wechselwirkungen der markierten Mineralöle mit gegebenenfalls vorliegenden anderen Inhaltsstoffen zu minimieren, strebt man an, die Menge an Markierstoffen möglichst gering zu halten. Ein weiterer Grund, die Menge an Markierstoffen möglichst klein zu halten, kann darin liegen, deren mögliche schädigende Einflüsse, beispielsweise auf den Kraftstoffein- und Abgasauslassbereich von Verbrennungsmotoren, zu unterbinden.

Wie weiter oben bereits erwähnt, ist man generell bestrebt, für die Markierung von Flüssigkeiten solche Verbindungen als Markierstoffe zu verwenden, welche eine hohe Fluoreszenzquantenausbeute ergeben, also einen großen Teil der absorbierten Strahlungsquanten als Fluoreszenzstrahlungsquanten wieder abgeben.

Diese als Markierstoffe zu verwendenden Verbindungen werden den Flüssigkeiten dabei in solchen Mengen zugegeben, daß eine zuverlässige Detektion gewährleistet ist. Üblicherweise beträgt der (gewichtbezogene) Gesamtgehalt an Markierstoffen in den markierten Flüssigkeit etwa 0,1 bis 5000 ppb, bevorzugt 1 bis 2000 ppb und besonders bevorzugt 1 bis 1000 ppb.

Zur Markierung der Flüssigkeiten werden die oben als Markierstoffe genannten Verbindungen (bzw. deren

Kombinationen aus mindestens zwei Markierstoffen) im allgemeinen in Form einer Lösung (Stammlösung) zugegeben. Insbesondere bei Mineralölen eignen sich als Lösungsmittel zur Bereitung dieser Stammlösungen vorzugsweise aromatische Kohlenwasserstoffe, wie Toluol, Xylol oder höhersiedende Aromatengemische.

Um eine zu hohe Viskosität solcher Stammlösungen (und damit schlechte Dosier- und Handhabbarkeit) zu vermeiden, wählt man im allgemeinen eine Gesamtkonzentration der Markierstoffe von 0,5 bis 50 Gew.-%, bezogen auf das Gesamtgewicht dieser Stammlösungen.

Folgendes sei hierzu definiert: der (die) zum Markierstoff Mμ gehörende(n) Wellenlängenbereich(e), in welchem (welchen) der Extinktionskoeffizient x% oder mehr des Wertes im Absorptionsmaximum des Markierstoffes Mμ beträgt, sei als "x-%-Wellenlängenintervall" Lμ (x) bezeichnet. Da beispielsweise die Anzahl n der Markierstoffe im Rahmen der bevorzugten Ausführungsformen 2 bis 10 bzw. 2 bis 6 bzw. 2 bis 4 beträgt, kann μ dementsprechend ganze Zahlenwerte von 1 bis 10 bzw. 1 bis 6 bzw. 1 bis 4 annehmen (entsprechend den Markierstoffen M1, M2, M3,..., M9, M10 bzw. M1, M2, M3, ..., M5, M6 bzw. M1, M2, M3, M4). Die entsprechenden Intervalle sind dann L1 (x), L2(x),..., L9(x), L10(x) bzw. L1 (x), L2(x),..., L5 (x), L6 (x) bzw. L1 (x), L2(x), L3(x), L4(x). Diese Intervalle sind in der Regel zusammenhängend, können jedoch auch unzusammenhängend sein. So kann ein spezielles Intervall Lμ (x) auch aus zwei oder mehreren Teilintervallen bestehen, wobei deren Gesamtheit (Vereinigungsmenge) dann als Lμ(x) bezeichnet wird.

Je nach Markierstoffen und/oder zu markierender Flüssigkeit kann man den Wert von x individuell wählen bzw. dadurch den Absorptionsbereich des Markierstoffs Mμ, in Abhängigkeit von den speziellen Erfordernissen, "definieren". So können, etwa bei hoher Absorption durch die zu markierende Flüssigkeit, Werte für x von z.B. 10, 20, 25 oder sogar 50 sinnvoll sein. Umgekehrt können bei Markierstoffen mit einem hohen Absorptionsvermögen und/oder zu markierenden Flüssigkeiten, welche im betrachteten Wellenlängenbereich nur geringe oder keine störenden Absorptionen zeigen, bereits Werte für x von z.B. 5 oder 3 ausreichend sein. Entsprechend diesen Werten für x werden dann beispielsweise durch L3(10) bzw. L3(20) bzw. L3(25) Wellenlängenintervalle bezeichnet, in welchen der Extinktionskoeffizient des Markierstoffs M3 mindestens 10 bzw. 20 bzw. 25% des Wertes im Absorptionsmaximum von M3 beträgt. Analog werden beispielsweise durch L3(5) bzw. L3(3) Wellenlängenintervalle definiert, in welchen der Extinktionskoeffizient des Markierstoffs M3 mindestens 5 bzw. 3% des Wertes im Absorptionsmaximum von M3 beträgt. Es kann sogar sinnvoll sein, innerhalb eines Ensembles von Markierstoffen verschiedene Werte von x zu verwenden. Vereinfachend wird im Folgenden anstelle von Lμ(x) (für das x-%-Intervall des Markierstoffs Mμ) lediglich Lμ geschrieben.

Weiter werden als Basis zur Definition der Intervalle Lμ(x) der verschiedenen, zur Markierung ins Auge gefaßten Markierstoffe Mμ deren jeweilige, unter vergleichbaren Bedingungen bestimmten Absorptionspektren herangezogen, welche hinsichtlich des Blindwerts des für die Bestimmung verwendeten Lösungsmittels korrigiert sind.

Entsprechend den obigen Ausführungen kann man das Wellenlängenintervall, in welchem das Absorptionsmaximum des Markierstoffs Mμ liegt, ebenfalls als Intervall Lμ (x) bezeichnen. Hier kann dann x, je nach Erfordernis, Werte von z.B. 80, 90, 95 oder auch 99 annehmen.

Für den beispielhaften Fall von sechs verschiedenen Markierstoffen M1, M2, M3, M4, M5 und M6, entsprechend einer bevorzugten Ausführungsform, in welcher die Zahl n der Markierstoffe den Wert sechs annimmt, ergeben sich die Intervalle L1, L2, L3, L4, L5 und L6. Die Überlappungsbereiche der Intervalle sind mathematisch gesehen deren Durchschnittsmengen und werden hier entsprechend als Lμν bezeichnet. Sie lassen sich systematisch aufstellen als:

$L1 \cap L2 = L12$ ($\mu = 1$, $\nu = 2$),
$L1 \cap L3 = L13$ ($\mu = 1$, $\nu = 3$),
$L1 \cap L4 = L14$ ($\mu = 1$, $\nu = 4$),
$L1 \cap L5 = L15$ ($\mu = 1$, $\nu = 5$),
$L1 \cap L6 = L16$ ($\mu = 1$, $\nu = 6$),
$L2 \cap L3 = L23$ ($\mu = 2$, $\nu = 3$),
$L2 \cap L4 = L24$ ($\mu = 2$, $\nu = 4$),
$L2 \cap L5 = L25$ ($\mu = 2$, $\nu = 5$),
$L2 \cap L6 = L26$ ($\mu= 2$, $\nu= 6$),
$L3 \cap L4 = L34$ ($\mu= 3$, $\nu= 4$),
$L3 \cap L5 = L35$ ($\mu= 3$, $\nu= 5$),
$L3 \cap L6 = L36$ ($\mu= 3$, $\nu= 6$),
$L4 \cap L5 = L45$ ($\mu = 4$, $\nu= 5$),
$L4 \cap L6 = L46$ ($\mu= 4$, $\nu= 6$) und
$L5 \cap L6 = L56$ ($\mu = 5$, $\nu= 6$).

Die Überlappungsbereiche der Intervalle Lμ mit sich selbst (Lμν mit $\nu = \mu$) kann man analog definieren als :

$L1 \cap L1 = L11 = L1$ ($\mu = 1$, $\nu= 1$),
$L2 \cap L2 = L22 = L2$ ($\mu = 2$, $\nu= 2$),

$L3 \cap L3 = L33 = L3$ ($\mu$= 3, $\nu$= 3),
$L4 \cap L4 = L44 = L4$ ($\mu$ = 4, $\nu$ = 4) ,
$L5 \cap L5 = L55 = L5$ ($\mu$ = 5, $\nu$= 5) und
$L6 \cap L6 = L66 = L6$ ($\mu$ = 6, $\nu$= 6).

Die Anzahl N der möglichen Überlappungsbereiche zwischen jeweils zwei Markierstoffen bei insgesamt n Markierstoffen berechnet sich allgemein zu N = n/2 • (n-1). Im vorliegenden Fall für n gleich sechs ergeben sich daher die oben aufgeführten 15 Überlappungsintervalle. Es sei hier angemerkt, daß die jeweiligen Überlappungsbereiche $L\mu\nu$ (z.B. L34) natürlich zu den Überlappungsbereichen $L\nu\mu$ (z.B. L43) äquivalent sind und daher nicht weiter betrachtet werden.

Gemäz dieser offenbarung soll der Absorptionsbereich mindestens eines Markierstoffes mit dem Absorptionsbereich mindestens eines weiteren Markierstoffs überlappen. Im Beispiel der sechs Markierstoffe M1 bis M6 bedeutet dies, daß mindestens einer der 15 Überlappungsbereiche verschieden von der leeren Menge sein muß.

Es lassen sich beispielsweise die folgenden Fälle diskutieren:

A) Alle benachbarten Wellenlängenintervalle $L\mu$ und L ($\mu$+1) bilden Überlappungsbereiche (Durchschnittsmengen), welche verschieden von der leeren Menge sind, alle nicht benachbarten Wellenlängenintervalle $L\mu$ und Lv (v > $\mu$ + 1) besitzen keine "höheren" Überlappungsbereiche (Durchschnittsmengen), bilden also jeweils leere Mengen, d.h.:

$$L\mu\nu \neq \{\varnothing\} \text{ für } \nu = \mu + 1 \text{ oder } \nu = \mu - 1$$

und

$$L\mu\nu = \{\varnothing\} \text{ für } \nu \neq \mu + 1 \text{ und } \nu \neq \mu - 1$$

("höhere" Überlappungsbereiche).

In obigem, beispielhaften Fall bedeutet dies, daß es nur Überlappungsbereiche L12, L23, L34, L45 und L56 (und natürlich Bereiche L11, L22, L33, L44, L55 und L 66 äquivalent zu den Intervallen L1, L2, L3, L4, L5 und L 6) gibt. Alle übrigen Überlappungsbereiche bilden leere Mengen. Durch Verwendung von fünf Strahlungsquellen, welche in diesen Überlappungsbereichen Strahlung emittieren, lassen sich daher alle sechs Markierstoffe M1 bis M6 zur Emission von Fluoreszenzstrahlung anregen, wobei jeweils durch eine Strahlungsquelle zwei Markierstoffe gleichzeitig angeregt werden. Allgemein werden also durch n-1 Strahlungsquellen, welche in den Bereichen L12, L23, ..., L(n-2)(n-1), L(n-1)n Strahlung emittieren, n Markierstoffe $M\mu$ angeregt. Weiter besitzen die Markierstoffe M1 und Mn (z.B. M6) jeweils nur einen (L12 bzw. L(n-1)n, z.B. L56), die übrigen Markierstoffe (z.B. M2 bis M5) jeweils zwei Überlappungsbereiche (z.B. besitzt $M\mu$ die Bereiche $L(\mu-1)\mu$ und $L\mu(\mu+1)$).

B)Mindestens ein Markierstoff $M\mu$ besitzt ein Wellenlängenintervall $L\mu$, welches mit dem Wellenlängenintervall L ($\mu$+2) des Markierstoffs M ($\mu$+2) einen "höheren" Überlappungsbereich $L\mu(\mu+2)$ bildet. Sofern dieser "höhere" Überlappungsbereich mit dem Wellenlängenintervall L ($\mu$+1) des Markierstoffs M ($\mu$+1) überlappt, was in der Regel der Fall ist, ergibt dies einen "dreifachen" Überlappungsbereich $L\mu$ ($\mu$+1) ($\mu$+2) der Intervalle $L\mu$, L($\mu$+1) und L ($\mu$+2). Durch Verwendung einer Strahlungsquelle, welche in diesem Bereich Strahlung emittiert, lassen sich daher die Markierstoffe $M\mu$, M($\mu$+1) und M($\mu$+2) gleichzeitig zur Emission von Fluoreszenzstrahlung anregen. Mathematisch läßt sich dies formulieren als:

$$L\mu\nu \neq \{\varnothing\} \text{ für } \nu = \mu + 2, \text{ d.h. } (L\mu(\mu + 2) = )\ L\mu \cap L(\mu + 2) \neq \{\varnothing\},$$

und

$$L(\mu + 1) \cap [L\mu \cap L(\mu + 2)] = L(\mu + 1) \cap L\mu \cap L(\mu + 2) = L\mu \cap L(\mu + 1) \cap L(\mu + 2) = L\mu(\mu + 1)(\mu + 2) \neq \{\varnothing\}.$$

In obigem, beispielhaften Fall für sechs Markierstoffe M1 bis M6 bedeutet dies, daß es z.B. einen Überlappungsbereich L13 gibt, welcher mit dem Intervall L2 (dem Überlappungsbereich L22) überlappt, also eine Durchschnittsmenge (L1 ∩ L3) ∩ L2 = L1 ∩ L3 ∩ L2 = L1 ∩ L2 ∩ L3 bildet, was sich, in Analogie zu den oben eingeführten Durchschnittsmengen zweier Intervalle, als Durchschnittsmenge L123 dreier Intervalle (oder allgemein als $L\mu\nu\omega$) abkürzen läßt. Weiter läßt sich auch unmittelbar erkennen, daß gilt:

$$L\mu\nu\omega = L\mu\omega\nu = L\nu\omega\mu = L\nu\mu\omega = L\omega\mu\nu = L\omega\nu\mu,$$

d.h. alle "permutierten" Bereiche sind zueinander äquivalent.

Durch Verwendung von z.B. drei Strahlungsquellen, welche in den Überlappungsbereichen L123, L45 und L56 Strahlung emittieren, lassen sich alle sechs Markierstoffe M1 bis M6 zur Emission von Fluoreszenzstrahlung anregen, wobei durch die Strahlungsquelle a(L123) gleichzeitig die Markierstoffe M1, M2 und M3 und durch die Strahlungsquelle a(L45) bzw. a(L56) gleichzeitig die Markierstoffe M4 und M5 bzw. M5 und M6 angeregt werden. Alternativ kann in letzterem Fall auch eine Strahlungsquelle verwendet werden, welche nicht im Bereich L56 sondern z.B. nur im Bereich L66 - L56, also dem Intervall L6 vermindert um den Überlappungsbereich (die Durchschnittsmenge) zwischen L5 und L6 Strahlung emittiert. Hierdurch erfolgt nur Anregung des Markierstoffs M6, jedoch keine Anregung des Markierstoffs M5.

Existiert zusätzlich ein Überlappungsbereich L456, so kann man bereits mit zwei Strahlungsquellen, welche jeweils in den Bereichen L123 bzw. L456 Strahlung emittieren, die Fluoreszenzen aller sechs Markierstoffe M1 bis M6 anregen, wobei jeweils die Anregung der Markierstoffe M1, M2 und M3 bzw. M4, M5 und M6 gleichzeitig erfolgt.

Im Folgenden seien mit $M\mu'$ solche Markierstoffe bezeichnet, welche untereinander keine Überlappungsbereiche zeigen, aber mit den Markierstoffen $M\mu$ vergleichbare Wellenlängen des jeweiligen Absorptionsmaximums aufweisen.

Für eine Mischung entsprechend dem oben diskutierten Fall A), d.h. aus sechs Markierstoffen M1 bis M6 mit den Bereichen L12, L23, L34, L45 und L56 (und natürlich den Bereichen L11, L22, L33, L44, L55 und L 66 äquivalent zu den Intervallen L1, L2, L3, L4, L5 und L 6) kann bereits mit fünf geeigneten Strahlungsquellen (diese seien mit a ($L\mu\nu$) bezeichnet, also a(L12), a(L23), a(L34), a(L45) und a(L56)) eine Anregung aller Markierstoffe erreicht werden. Im Gegensatz dazu kann eine Mischung von sechs Markierstoffen M1', M2', M3', M4', M5' und M6' mit den Strahlungsquellen a(L12) bis a(L56) nicht zur Emission von Fluoreszenzstrahlung angeregt werden. Eine solche Mischung entspricht einer Mischung, wie sie durch den Stand der Technik, z.B. in der Schrift US 5,525,516, nahegelegt wird.

Ähnliches gilt für den oben diskutierten Fall B). Hier wird durch eine Strahlungsquelle a(L123) eine Anregung der Markierstoffe M1, M2 und M3 erreicht. Im Falle der Markierstoffe M1', M2' und M3' könnte lediglich eine Anregung des Markierstoffs M2' erfolgen, wenn dessen Wellenlänge des Absorptionsmaximums im Bereich L123 liegt. Die Markierstoffe M1' und M3' würden jedoch durch eine solche Strahlungsquelle nicht zur Emission von Fluoreszenzstrahlung angeregt.

Weiter kann man eine Markierung von Flüssigkeiten in der Art vornehmen, daß man z.B. Markierstoffe M1 bis M6 verwendet, welche Überlappungsbereiche L123, L34, L55 (= L5) und L66 (= L6) besitzen. Durch die Strahlungsquellen a(L123), a(L34), a(L55) und a(L66) werden alle Markierstoffe angeregt. Im Gegensatz dazu wird im Falle der Markierstoffe M1' bis M6' durch diese Strahlungsquellen nur eine Anregung der Markierstoffe M5', M6' und gegebenenfalls M2', jedoch nicht der Markierstoffe M1', M3' und M4' erreicht.

Weiter kann man z.B. eine Mischung von Markierstoffen M1 und M2 verwenden, welche (definitionsgemäß) einen Überlappungsbereich L12 besitzt. Fällt beispielsweise das Wellenlängenintervall des Absorptionsmaximums des Markierstoffs M1 (je nach Erfordernis kann dieser "Hauptabsorptionsbereich" z.B. als L1(80), L1(90), L1(95) oder L1 (99) definiert werden, s.o.) in den Bereich L12, so kann man zwei Strahlungsquellen a(L1) und a(L2) verwenden, welche im Bereich der jeweiligen Absorptionsmaxima (bzw. der jeweiligen "Hauptabsorptionsbereiche") der Markierstoffe M1 und M2 (z.B. L1(80), L1(90), L1 (95) oder L1 (99) bzw. L2(80), L2(90), L2(95) oder L2 (99)) Strahlung emittieren. Die Intensität der durch M1 emittierten Fluoreszenzstrahlung wird dabei vollständig durch die von der Strahlungsquelle a (L1) absorbierte Strahlungsintensität bestimmt. Dagegen resultiert die Intensität der durch M2 emittierten Fluoreszenzstrahlung aus den Anteilen der durch M2 von der Strahlungsquelle a(L2) und der Strahlungsquelle a(L1) im Überlappungsbereich L12 absorbierten Strahlungs-intensität. Im Falle einer Mischung aus M1' und M2' wird die jeweilige Intensität der durch M1' bzw. M2' emittierten Fluoreszenzstrahlung vollständig durch die von der Strahlungsquelle a (L1) bzw. a(L2) absorbierte Strahlungsintensität bestimmt. Eine solche Mischung aus M1' und M2' entspricht einer Mischung, wie sie durch den Stand der Technik, z.B. in der Schrift US 5,525,516, nahegelegt wird.

Weiter kann man eine Mischung von Markierstoffen entsprechend den Fälle A) und/oder B) mit solchen Markierstoffen, welche keine Überlappungsbereiche mit den übrigen Markierstoffen besitzen, verwenden. Als weiteren Schutz vor Nachahmung kann man auch den Nachweis der Markierstoffe durch Detektion der mit verschiedenen, festgelegten Kombinationen von Strahlungsquellen angeregten Floureszenzstrahlung durchführen. Dies sei im Folgenden beispielhaft ausgeführt ohne jedoch eine Einschränkung zu implizieren.

Beispiel 1: Die Markierstoffe M1 bis M4 besitzen die Überlappungsbereiche L12, L23 und L34. Die Wellenlängenintervalle L1 (x), L2(x), L3(x) und L4(x), in welchen die jeweiligen Absorptionsmaxima der Markierstoffe M1 bis M4 liegen (diese "Hauptabsorptionsbereiche" lassen sich z.B. als $L\mu(x)$ mit x gleich z.B. 80, 90, 95 oder 99(%) definieren, s.o.) besitzen keinen Überlappungsbereich (keine Durchschnittsmenge) mit den Bereichen L12, L23 und L34.

Kombination 1.1: Es werden die Strahlungsquellen a(L1(x)), a (L2(x)), a (L3(x)) und a (L4(x)) verwendet. Die Markierstoffe emittieren ihre jeweilige Fluoreszenzstrahlung. Es findet keine kombinierte Anregung von Fluoreszenz statt.

Kombination 1.2: Es werden die Strahlungsquellen a(L12), a(L23) und a(L34) verwendet. Die Markierstoffe emittieren ihre jeweilige Fluoreszenzstrahlung aufgrund der kombinierten Anregung. Durch a(L12) werden die Markierstoffe M1 und M2, durch a(L23) die Markierstoffe M2 und M3 und durch a(L34) die Markierstoffe M3 und M4 zur Emission von Fluoreszenz angeregt. Erfolgt die Anregung der Markierstoffe M1 bis M4 durch die Strahlungsquellen gleichzeitig, so tragen sowohl die Anregung durch a(L12) und a(L23) bzw. durch a(L23) und a(L34) zur Fluoreszenzintensität des Markierstoffs M2 bzw. M3 bei. Wird die Anregung der Markierstoffe durch die Strahlungsquellen nacheinander durchgeführt, so erhält man jeweils Teilspektren der emittierten Fluoreszenzstrahlung, welche eine vom ersteren Fall abweichende Intensitätsverteilung der durch die Markierstoffe M2 und M3 emittierten Fluoreszenzstrahlung zeigen. Diese (drei) Teilspektren lassen sich aber (z.B. rechnerisch mit Hilfe entsprechender Computerprogramme) zum Gesamtspektrum des ersteren Falles zusammensetzen. Es ist im Regelfall zu erwarten, daß die Intensitätsverteilungen der Fluoreszenzen der Markierstoffe M1 bis M4 im Falle der Kombination 1.2 (kombinierte Anregung) von den Intensitätsverteilungen der Kombination 1.1 abweichen. Durch die Abfolge der Detektionen entsprechend den Kombinationen 1.1 und 1.2 wird daher ein "doppelter Fingerabdruck" der Markierstoff-Mischung bzw. der Flüssigkeit, enthaltend eine solche Markierstoff-Mischung, erzeugt. Natürlich lassen sich auch die in den Kombinationen 1.1 und 1.2 beschriebenen Strahlungsquellen miteinander kombinieren.

Beispiel 2: Die Markierstoffe M1 bis M4 besitzen die Überlappungsbereiche L12, L23 und L34. Die Wellenlängenintervalle L1(x), L2 (x), L3(x) und L4(x), in welchen die jeweiligen Absorptionsmaxima der Markierstoffe M1 bis M4 liegen (diese "Hauptabsorptionsbereiche" lassen sich z.B. als $L\mu$ (x) mit x gleich z.B. 80, 90, 95 oder 99(%) definieren, s.o.) besitzen Überlappungsbereiche (Durchschnittsmengen) mit den Bereichen L12, L23 und L34, z.B. sei $L12 \cap L1(x) \neq \{\varnothing\}$, $L23 \cap L2(x) \neq \{\varnothing\}$, $L23 \cap L3(x) \neq \{\varnothing\}$ und $L34 \cap L4(x) \neq \{\varnothing\}$ .

Kombination 2.1: Es werden die Strahlungsquellen a(L1(x)), a(L2 (x)), a(L3(x)) und a(L4(x)) verwendet. Die Markierstoffe emittieren ihre jeweilige Fluoreszenzstrahlung aufgrund der kombinierten Anregung. Durch a(L1(x)) werden die Markierstoffe M1 und M2, durch a(L2(x)) und a(L3(x)) die Markierstoffe M2 und M3 und durch a(L4(x)) die Markierstoffe M3 und M4 zur Emission von Fluoreszenzstrahlung angeregt.

Kombination 2.2: Es werden die Strahlungsquellen a(L1(x)), a(L3(x)) und a(L4(x)) verwendet. Die Markierstoffe emittieren ihre jeweilige Fluoreszenzstrahlung aufgrund der kombinierten Anregung. Durch a(L1(x)) werden die Markierstoffe M1 und M2, durch a(L3(x)) die Markierstoffe M2 und M3 und durch a(L4(x)) die Markierstoffe M3 und M4 zur Emission von Fluoreszenzstrahlung angeregt. Hinsichtlich der gleichzeitigen bzw. zeitlich aufeinander folgenden Anregung durch die Strahlungsquellen gilt auch hier das bereits unter Beispiel 1, Kombination 1.2 Gesagte. Die Intensitätsverhältnisse unterscheiden sich jedoch von jenen aus Kombination 2.1.

5 Kombination 2.3: Es werden die Strahlungsquellen a(L12), a(L23) und a(L34) verwendet. Die Markierstoffe emittieren ihre jeweilige Fluoreszenzstrahlung aufgrund der kombinierten Anregung. Durch a(L12) werden die Markierstoffe M1 und M2, durch a(L23) die Markierstoffe M2 und M3 und durch a(L34) die Markierstoffe M3 und M4 zur Emission von Fluoreszenz angeregt. Hinsichtlich der gleichzeitigen bzw. zeitlich aufeinander folgenden Anregung durch die Strahlungsquellen gilt auch hier das bereits unter Beispiel 1, Kombination 1.2 Gesagte. Es ist im Regelfall zu erwarten, daß die Intensitätsverteilungen der Fluoreszenzen der Markierstoffe M1 bis M4 im Falle der Kombination 2.3 von den Intensitätsverteilungen der Kombinationen 2.1 und 2.2 abweichen. Durch die Abfolge der Detektionen entsprechend den Kombinationen 2.1, 2.2 und 2.3 wird daher ein "dreifacher Fingerabdruck" der Markierstoff-Mischung bzw. der Flüssigkeit, enthaltend eine solche Markierstoff-Mischung, erzeugt. Natürlich lassen sich auch hier die in den Kombinationen 2.1, 2.2 und 2.3 beschriebenen Strahlungsquellen miteinander kombinieren.

Zur Markierung von Flüssigkeiten ist es selbstverständlich auch möglich, Kombinationen von Markierstoffen zu verwenden, welche sich in den relativen Mengen der Markierstoffe zueinander unterscheiden. So kann beispielsweise die Markierung einer Flüssigkeit mit einer Mischung der Markierstoffe M1 bis M4 im Molverhältnis M1:M2:M3:M4 = 1:1:1:1, die einer anderen Flüssigkeit mit einer Mischung im Verhältnis von z.B. 2:1:1:1, 4:1:1:1, 8:1:1:1, 2:2:1:1, 2:4:1:1, 2:8:1:1, 4:4:1:1, 4:8:1:1, 8:8:1:1, 2:2:2:1, 2:2:4:1, 2:2:8:1, 2:2:2:2, 2:2:2:4, 2:2:2:8 oder auch im Verhältnis einer entsprechenden Permutation davon erfolgen.

Beabsichtigt man, verschiedene Flüssigkeiten nur durch unterschiedliche Konzentrationen einer festgelegten Mischung von Markierstoffen (z.B. M1 bis M4) zu markieren, so wird man üblicherweise die jeweilige Konzentration so wählen, daß sie sich von Flüssigkeit zu Flüssigkeit mindestens um den Faktor zwei unterscheidet, um die Eindeutigkeit des Nachweises zu gewährleisten. Bevorzugt ist zur Markierung verschiedener Flüssigkeiten jedoch die Verwendung

von Markierstoff-Mischungen unterschiedlicher molarer Verhältnisse der Markierstoffe zueinander.

Der prinzipielle apparative Aufbau zur Anregung und zum Nachweis der Fluoreszenz in eine markierten Flüssigkeit enthält:

eine Probenküvette, welche die markierte Flüssigkeit enthält,

eine Anregungseinheit (A), welche enthält:

$\alpha_1$) eine Strahlungsquelle, welche üblicherweise mit einer Kollimatoroptik versehen ist, und

$\alpha_2$) üblicherweise einen Planspiegel, welcher sich der Strahlungsquelle gegenüber auf der, der Strahlungsquelle abgewandten Seite der Probenküvette befindet und durch Reflektion der transmittierten Strahlung der Erhöhung der in die Probe eingestrahlten Intensität dient,

eine Detektionseinheit (D), welche enthält:

$\delta_1$) einen Photodetektor (üblicherweise mit einer Kollimatoroptik versehen), vor welchem sich üblicherweise optische Filter (z.B. Kanten- oder Interferenzfilter) und gegebenenfalls NIR-Polarisatoren befinden und welcher so angeordnet ist, daß die in seine Richtung emittierte Fluoreszenzstrahlung auf ihn auftrifft (oder abgebildet wird) und nachgewiesen wird, und

$\delta_2$) üblicherweise einen Hohlspiegel, welcher sich dem Photodetektor gegenüber auf der, dem Photodetektor abgewandten Seite der Probenküvette befindet, und durch Reflektion der in Gegenrichtung (vom Photodetektor weg) emittierten Fluoreszenzstrahlung, und damit der Erhöhung der Nachweisempfindlichkeit, dient.

Ein solcher Aufbau ist im Prinzip in der Schrift WO 94/02570 zeichnerisch dargestellt (abweichend zur Strahlrichtung der Strahlungsquelle). Die Detektion der Fluoreszenzstrahlung muß dabei aber nicht senkrecht, sondern kann unter nahezu beliebigem Winkel zur Strahlrichtung erfolgen. Sinnvollerweise kommen aber Winkel von 0° bzw. 180° nicht in Betracht.

Im Hinblick auf die nachfolgenden Ausführungen sollen folgende Definitionen gelten:

Anregungs- bzw. Detektionseinheiten im allgemeinen Sinn werden als A bzw. D bezeichnet (s.o). Markierstoffe im allgemeinen Sinn werden als M bezeichnet.

Eine Anregungseinheit, welche mittels einer entsprechenden Strahlungsquelle speziell im Wellenlängenintervall $L\mu$ (synonym zum "Überlappungsbereich" $L\mu\mu$ oder $L\mu\mu\mu$), dem Überlappungsbereich $L\mu\nu$ oder dem Überlappungsbereich $L\mu\nu\omega$ eingestellt ist (d.h. die Strahlungsquelle emittiert Strahlung im Wellenlängenintervall $L\mu$ des Markierstoffs $M\mu$, im Überlappungsbereich der Markierstoffe $M\mu$ und $M\nu$ oder im Überlappungsbereich der Markierstoffe $M\mu$, $M\nu$ und $M\omega$), wird einheitlich mit $A(L\mu\nu\omega)$ oder kürzer mit $A\mu\nu\omega$ bezeichnet bzw. verschiedene solcher Einheiten werden mit $A\mu_1\nu_1\omega_1,..., A\mu_n\nu_n\omega_n$ bezeichnet, wobei beispielsweise für n gleich 2 bis 10 bzw. 2 bis 6 bzw. 2 bis 4 die Parameter $\mu$, $\nu$, und $\omega$ (bzw. $\mu_1$, ..., $\mu_n$, $\nu_1$, ..., $\nu_n$ und $\omega_1,...,\omega_n$) jeweils werte von 1 bis 10 bzw. 1 bis 6 bzw. 1 bis 4 annehmen können. Hierbei sind natürlich mehrere der Einheiten $A\mu\nu\omega$ aufgrund der Definition von $L\mu\nu\omega$ äquivalent, was bereits weiter oben erwähnt wurde. Beispielsweise sind durch die Definition von L123 als $L1 \cap L2 \cap L3$ ($= L3 \cap L1 \cap L2 = L2 \cap L3 \cap L1 = L2 \cap L1 \cap L3 = L1 \cap L3 \cap L2 = L3 \cap L2 \cap L1$) die Einheiten A123 ($\mu_1$=1, $\nu_2$=2 und $\omega_3$=3), A312 ($\mu_3$=3, $\nu_1$=1 und $\omega_2$=2), A231 ($\mu_2$=2, $\nu_3$=3 und $\omega_1$=1), A213 ($\mu_2$=2, $\nu_1$=1 und $\omega_3$=3), A132 ($\mu_1$=1, $\nu_3$=3 und $\omega_2$=2) und A321 ($\mu_3$=3, $\nu_2$=2 und $\omega_1$=1) identisch. Diese weiteren, zu einer Einheit identischen Einheiten sollen nicht weiter berücksichtigt werden, wie dies bereits im Falle der äquivalenten Überlappungsbereiche $L\mu\nu$ und $L\nu\mu$ bei der eingangs gegebenen Definition der Überlappungsbereiche geschehen ist.

Natürlich sind auch höhere "Überlappungsbereiche", z.B. $L\mu\nu\omega\chi$ usw. bzw. entsprechende Strahlungsquellen $A\mu\nu\omega\chi$ usw. möglich. Diese sollen hier jedoch nicht weiter berücksichtigt werden.

$A\mu$, $A\mu\mu$ und $A\mu\mu\mu$ sind als synonyme Schreibweisen zu betrachten und bezeichnen identische Anregungseinheiten, welche im Wellenlängenintervall $L\mu$ ($= L\mu\mu = L\mu\mu\mu$) des Markierstoffs $M\mu$ Strahlung emittieren.

Eine spezielle Detektionseinheit, welche, z.B. mittels entsprechender optischer Filter (und gegebenenfalls Polarisatoren), speziell auf die emittierte Fluoreszenzstrahlung eines der Märkierstoffe $M\mu$ eingestellt ist, wird mit $D\mu$ (oder auch als Detektionskanal $\mu$) bezeichnet.

So lassen sich z.B. den drei Markierstoffen M1, M2 und M3 die Anregungseinheiten A1 (= A11 = A111), A2 (= A22 = A222), A3 (= A33 = A333), A12 (= A112 = A122), A13 (= A113 = A133), A23 (= A223 = A233) und A123 bzw. die daran angepaßten Detektionseinheiten D1, D2 und D3 zuordnen.

Weiter kann auch für z.B. zwei Markierstoffe M1 und M2 (wenn ein Überlappungsbereich L12 existiert) die Anregung der Fluoreszenzstrahlung der Markierstoffe M1 und M2 durch die identische Einheit A12 erfolgen. Die Detektion

der Fluoreszenzstrahlung geschieht dann mittels der jeweiligen Einheiten D1 und D2. Die Kombinationen aus Anregung und Detektion lassen sich dann als A12/D1 und A12/D2 schreiben.

Findet innerhalb einer Anregungseinheit A die Anpassung an den jeweiligen Überlappungsbereich $L\mu\nu\omega$ lediglich durch den Einsatz einer entsprechenden Strahlungsquelle $\alpha_1\mu\nu\omega$ statt (d.h. diese emittiert Strahlung im Wellenlängenintervall $L\mu$ des Markierstoffs $M\mu$, im Überlappungsbereich der Markierstoffe $M\mu$ und Mv oder im Überlappungsbereich der Markierstoffe $M\mu$, Mv und $M\omega$), so wird dies, für den Fall von n Markierstoffen, mit $A(\mu_1\nu_1\omega_1, ..., \mu_n\nu_n\omega_n)$ bezeichnet, wobei beispielsweise für n gleich 2 bis 10 bzw. 2 bis 6 bzw. 2 bis 4 die Parameter $\mu_1,..., \mu_n$, $\nu_1,...,\nu_n$ und $\omega_1,...,\omega_n$ jeweils Werte von 1 bis 10 bzw. 1 bis 6 bzw. 1 bis 4 annehmen können. Beispielsweise bezeichnet A(111, 112, 223) (= A(1, 12, 23)) eine Anregungseinheit, welche durch den Einsatz der Strahlungsquellen $\alpha_1$111 (= $\alpha_1$1), $\alpha_1$112 (= $\alpha_1$12) und $\alpha_1$223 (= $\alpha_1$23) an das Wellenlängenintervall L1 (den Überlappungsbereich L111) des Markierstoffs M1 und die Überlappungsbereiche L12 bzw. L23 der Markierstoffe M1 und M2 bzw. M2 und M3 angepaßt werden kann.

Für eine Detektionseinheit $D\mu$, in welcher die Anpassung an die emittierte Fluoreszenzstrahlung des jeweiligen Markierstoffs $M\mu$ lediglich durch den Einsatz entsprechender Photodetektoren und/oder optischer Filter (und gegebenenfalls Polarisatoren) erfolgt, gilt für den Fall von n Markierstoffen die Bezeichnungen D(1,2), D(1,2,3), usw. bis D (1,2,3,...,9,10) bzw. D(1,2), D(1,2,3) usw. bis D(1,2,...,5,6) bzw. D (1,2), D(1,2,3), D(1,2,3,4), wobei hier beispielsweise n wiederum gleich 2 bis 10 bzw. 2 bis 6 ist bzw. 2 bis 4 ist.

Werden die Überlappungsbereiche $L\mu\nu\omega$ der n Markierstoff $M\mu$, wobei n beispielsweise wieder gleich 2 bis 10 bzw. 2 bis 6 bzw. 2 bis 4 ist, jeweils nacheinander mit den jeweils an sie angepassten Einheiten $A\mu\nu\omega$ angeregt und die vom jeweiligen Markierstoff $M\mu$ emittierte Fluoreszenzstrahlung durch $D\mu$ detektiert, so wird dies durch die Schreibweise "$A\mu_1\nu_1\omega_1$/D1, $A\mu_2\nu_2\omega_2$/D2,..." usw. bis "...$A\mu_9\nu_9\omega_9$/D9, $A\mu_{10}\nu_{10}\omega_{10}$/D10" bzw. "$A\mu_1\nu_1\omega_1$/D1, $A\mu_2\nu_2\omega_2$/D2,..." usw. bis "...$A\mu_5\nu_5\omega_5$/D5, $A\mu_6\nu_6\omega_6$/D6" bzw. "$A\mu_1\nu_1\omega_1$/D1, $A\mu_2\nu_2\omega_2$/D2, $A\mu_3\nu_3\omega_3$/D3, $A\mu_4\nu_4\omega_4$/D4" zum Ausdruck gebracht.

Werden die Überlappungsbereiche $L\mu\nu\omega$ der n Markierstoffe $M\mu$ gleichzeitig mit einer entsprechenden Anzahl von Einheiten $A\mu\nu\omega$ angeregt und gleichzeitig deren emittierte Fluoreszenzstrahlungen mit n Einheiten $D\mu$ detektiert so, wird dies mit "$A\mu_1\nu_1\omega_1$/$A\mu_2\nu_2\omega_2$/.../$A\mu_n\nu_n\omega_n$/D1/D2/.../Dn" bezeichnet.

Anmerkung: entsprechend dem oben Gesagten können n oder auch weniger Einheiten $A\mu\nu\omega$ vorliegen. Zur Vereinfachung wird jedoch "$A\mu_1\nu_1\omega_1$/$A\mu_2\nu_2\omega_2$/.../$A\mu_n\nu_n\omega_n$/" geschrieben.

Werden die Überlappungsbereiche $L\mu\nu\omega$ der n Markierstoffe gleichzeitig mit einer entsprechenden Anzahl von Einheiten $A\mu\nu\omega$ angeregt und die von den Markierstoffen $M\mu$ emittierte Fluoreszenzstrahlung mit einer Einheit D, z.B. einem Vielwellenlängendetektor (bestehend aus einem optischen, dispersiven Element, wie z.B. einem Prisma oder Gitter, und einem Zeilen- oder Flächendetektor), detektiert, so wird dies mit "$A\mu_1\nu_1\omega_1$/$A\mu_2\nu_2\omega_2$/ ... /$A\mu_n\nu_n\omega_n$/D" bezeichnet.

Im wesentlichen sind die beiden Fälle zu unterscheiden, daß die Anregung der markierten Probe durch die Einheiten A

I) in demselben Probenvolumen oder
II) in verschiedenen Probenvolumina erfolgt.

In Fall I) können folgende Verfahren und beispielhafte Aufbaumöglichkeiten der Detektionsgeräte Anwendung finden (n nimmt hierbei z.B. die Werte von 2 bis 10 bzw. 2 bis 6 bzw. 2 bis 4 an):

I.1) $A\mu_1\nu_1\omega_1$/D1, $A\mu_2\nu_2\omega_2$/D2, ... , $A\mu_{n-1}\nu_{n-1}\omega_{n-1}$/Dn-1 , $A\mu_n\nu_n\omega_n$/Dn (die n Markierstoffe $M\mu$ werden jeweils in den Überlappungsbereichen $L\mu\nu\omega$ durch ihre entsprechenden Einheiten $A\mu\nu\omega$ angeregt bzw. die Fluoreszenzen der Markierstoffe $M\mu$ jeweils durch die Einheiten $D\mu$ detektiert).

a) Der Aufbau entspricht im wesentlichen dem eingangs erwähnten und in der Schrift WO 94/02570 gezeigten Aufbau. Der Unterschied besteht darin, daß für jeden Markierstoff $M\mu$ jeweils eine entsprechende Einheit $A\mu\nu\omega$ bzw. $D\mu$ verwendet werden. Dies kann durch die räumlich versetzte Anordnung mehrerer, der Anzahl der zu detektierenden Markierstoffe entsprechenden Paare von Einheiten $A\mu\nu\omega$ und $D\mu$ radial um die Probenküvette erfolgen. Letztere besitzt dann vorzugsweise einen kreisförmigen Querschnitt. Die von den Einheiten $A\mu\nu\omega$ durchstrahlten Probenvolumina (bzw. Probenwege) sind dabei jedoch -im strengen Sinne- nicht identisch. Die (in einer Ebene liegenden) Anregungsstrahlen schneiden sich jedoch in einem gemeinsamen Stück des Probenvolumens. Mehrere Einheiten $A\mu\nu\omega$ können dabei identisch sein (und sind es in der Regel auch). So kann man z.B. bei drei Markierstoffen M1 bis M3 (z.B. mit den Überlappungsbereichen L12 und L23) zur Anregung und Detektion die entsprechenden (drei) Paare A12/D1, A12/D2 und A23/D3 oder auch A12/D1, A23/D2 und A23/D3 verwenden. Die Kombination von Anregung und Detektion der n Markierstoffe $M\mu$ kann sowohl nacheinander als auch gleichzeitig erfolgen.

b) Der Aufbau entspricht im wesentlichen dem eingangs erwähnten und in der Schrift WO 94/02570 gezeigten

Aufbau. Der Unterschied besteht darin, daß sich die Strahlungsquellen $\alpha_1\mu\nu\omega$ bzw. Photodetektoren $\delta_1\mu$ der Einheiten $A\mu\nu\omega$ bzw. $D\mu$ jeweils auf entsprechenden Karussells befinden (anstelle von individuellen Planspiegeln $\alpha_2\mu\nu\omega$ bzw. Hohlspiegeln $\delta_2\mu$ verwendet man in diesem Fall sinnvollerweise nur jeweils einen feststehenden Plan- bzw. Hohlspiegel). Zur Detektion des Markierstoffs $M\mu$ wird die entsprechende Strahlungsquelle $\alpha_1\mu\nu\omega$ bzw. der entsprechende Photodetektor $\delta_1\mu$ durch Rotation der jeweiligen Karussells in die Anregungs- bzw. Detektionsposition bewegt. Der Strahlenverlauf durch die markierte Probe und das durchstrahlte Probenvolumen sind für jeden zu bestimmenden Markierstoff identisch. Die Kombination von Anregung und Detektion der n Markierstoffe $M\mu$ kann nur nacheinander erfolgen.

c) Verwendet man beispielsweise eine zylinderförmige Probenküvette, welche entweder an einem oder an beiden Enden mit Fenstern aus demselben Material, aus welchem die Küvette besteht, verschlossen werden kann oder welche an beiden Enden geschlossen ist und vorzugsweise seitliche Probenein- und -auslässe besitzt, so läßt sich ein geänderter Aufbau erstellen. Ähnlich wie unter a) beschrieben können sich die Strahlungsquellen $\alpha_1\mu\nu\omega$ der Einheiten $A\mu\nu\omega$ auf einem Karussell befinden (anstelle von individuellen Planspiegeln $\alpha_2\mu\nu\omega$ verwendet man dann sinnvollerweise nur einen feststehenden Planspiegel). Die jeweilige Einheit $A\mu\nu\omega$ strahlt dann parallel zur Längsachse der Küvette ein. Radial dazu (und damit immer senkrecht zur Strahlrichtung der Anregungsstrahlung) lassen sich die jeweiligen Einheiten $D\mu$ (diesmal selbstverständlich mit ihren jeweiligen Untereinheiten $\delta_2\mu\nu\omega$) zur Detektion der jeweils emittierten Fluoreszenzstrahlung um die Probenküvette plazieren. Die Kombination von Anregung und Detektion der n Markierstoffe $M\mu$ kann nur nacheinander erfolgen (potentiell kann natürlich die Detektion der von mehreren Markierstoffen gleichzeitig emittierten Fluoreszenzstrahlung durch die Einheiten $D\mu$ simultan erfolgen).

I.2) A/D1,A/D2,...,A/Dn-1,A/Dn (alle n Markierstoffe $M\mu$ werden gleichzeitig durch eine "polychromatische" Strahlungsquelle angeregt und mittels ihres jeweiligen Detektionskanals $D\mu$ detektiert).

a) Der Aufbau kann ähnlich dem in b) unter I.1) beschriebenen erfolgen. Anstelle eines entsprechenden Karussells für die Untereinheiten $\alpha_1\mu\nu\omega$ wird jedoch nur eine polychromatische Anregungseinheit A verwendet. Die Detektion erfolgt entsprechend dem unter b) von I.1) beschriebenen Aufbau. Die Kombination von Anregung und Detektion der n Markierstoffe $M\mu$ kann nur nacheinander erfolgen.

b) Verwendet man, in Anlehnung an den in c) unter I.1) beschriebenen Aufbau, beispielsweise eine zylinderförmige Probenküvette, so strahlt die Einheit A parallel zur Längsachse der Küvette ein. Radial dazu (und damit jeweils senkrecht zur Strahlrichtung der Anregungsstrahlung) lassen sich wiederum die jeweiligen Einheiten $D\mu$ um die Probenküvette plazieren. Die Kombination von Anregung und Detektion der n Markierstoffe $M\mu$ kann sowohl nacheinander als auch gleichzeitig erfolgen.

I.3) $A\mu_1\nu_1\omega_1$/D, $A\mu_2\nu_2\omega_2$/D,..., $A\mu_n$-$1\nu_n$-$1\omega_n$-1/D, $A\mu_n\nu_n\omega_n$/D (die n Markierstoffe $M\mu$ werden jeweils in den Überlappungsbereichen $L\mu\nu\omega$ durch die entsprechenden Einheiten $A\mu\nu\omega$ angeregt bzw. die Fluoreszenzen der Markierstoffe $M\mu$ jeweils durch die Einheiten $D\mu$ detektiert).

a) Der Aufbau kann ähnlich dem in b) unter I.1) beschriebenen erfolgen. Anstelle eines entsprechenden Karussells für die Photodetektoren $\delta_1\mu$ wird jedoch nur eine Detektionseinheit D, z.B. ein Vielwellenlängendetektor, verwendet. Entsprechend dem unter b) von I.1) beschriebenen Aufbau erfolgt die Anregung. Die Kombination von Anregung und Detektion der n Markierstoffe $M\mu$ kann nur nacheinander erfolgen.

b) Verwendet man, in Anlehnung an den in c) unter I.1) beschriebenen Aufbau, beispielsweise eine zylinderförmige Probenküvette, so detektiert die Einheit D die jeweils emittierte Fluoreszenzstrahlung parallel zur Längsachse der Küvette. Radial dazu (und damit jeweils senkrecht zur Längsachse der Küvette) lassen sich die jeweiligen Einheiten $A\mu\nu\omega$ (zusammen mit ihren entsprechenden Planspiegeln $\alpha_2\mu\nu\omega$) um die Probenküvette plazieren. Die Kombination von Anregung und Detektion der n Markierstoffe $M\mu$ kann sowohl nacheinander als auch gleichzeitig erfolgen.

Die hier genannten Möglichkeiten des Aufbaus sind damit den in a) und b) von Punkt I.2) genannten vergleichbar und unterscheiden sich nur durch den räumlichen Austausch der Anregungs- und Detektionseinheit(en).

I.4) A

$$A(\mu_1\nu_1\omega_1, \mu_2\nu_2\omega_2, ..., \mu_{n-1}\nu_{n-1}\omega_{n-1}, \mu_n\nu_n\omega_n)/D1,$$

$$A(\mu_1\nu_1\omega_1, \mu_2\nu_2\omega_2, ..., \mu_{n-1}\nu_{n-1}\omega_{n-1}, \mu_n\nu_n\omega_n)/D2, ...,$$

$$A(\mu_1\nu_1\omega_1, \mu_2\nu_2\omega_2, ..., \mu_{n-1}\nu_{n-1}\omega_{n-1}, \mu_n\nu_n\omega_n)/Dn-1,$$

$$A(\mu_1\nu_1\omega_1, \mu_2\nu_2\omega_2, ..., \mu_{n-1}\nu_{n-1}\omega_{n-1}, \mu_n\nu_n\omega_n)/Dn$$

(die n Markierstoffe $M\mu$ werden jeweils in den Überlappungsbereichen $L\mu\nu\omega$ angeregt und mittels ihres jeweiligen Detektionskanals $D\mu$ detektiert).

a) Der Aufbau kann ähnlich dem in a) unter I.2) beschriebenen erfolgen. Anstelle eines entsprechenden Karussells für die Strahlungsquellen $\alpha_1\mu$ wird jedoch eine Anregungseinheit A verwendet, welche z.B. (Fall $a_1$) austauschbare Strahlungsquellen $\alpha_1\mu\nu\omega$ enthält. Weiter kann A aber auch mehrere Strahlungsquellen $\alpha_1\mu\nu\omega$ enthalten, deren jeweilige Strahlung, z.B. (Fall $a_2$) mittels Lichtleitfasern oder Lichtleitfaserbündeln oder kollinearer Überlagerung der einzelnen Strahlen der Strahlungsquellen mittels optischer Elemente, wie z.B. Strahlteiler, dichroitische Strahlteiler, Gitter usw., so geleitet wird, daß sie jeweils am selben Ort der Probenküvette in diese eintritt. Entsprechend dem unter a) von 1.2) beschriebenen Aufbau erfolgt die Detektion der jeweiligen Fluoreszenzstrahlung. Die Kombination von Anregung und Detektion der n Markierstoffe $M\mu$ kann nur nacheinander erfolgen.

b) verwendet man, in Anlehnung an den in c) unter I.1) beschriebenen Aufbau, beispielsweise eine zylinderförmige Probenküvette, so strahlt eine, wie unter a) (Fall $a_1$ oder Fall $a_2$) beschriebene Einheit A parallel zur Längsachse der Küvette ein. Radial dazu (und damit jeweils senkrecht zur Strahlrichtung der Anregungsstrahlung) lassen sich wiederum die jeweiligen Einheiten $D\mu$ um die Probenküvette plazieren. Mit einer Einheit A entsprechend Fall $a_1$ kann die Kombination von Anregung und Detektion der n Markierstoffe $M\mu$ nur nacheinander erfolgen. Mit einer Einheit A entsprechend Fall $a_2$ kann die Kombination von Anregung und Detektion der n Markierstoffe $M\mu$ nacheinander und potentiell auch gleichzeitig erfolgen.

I.5) $A\mu_1\nu_1\omega_1/D(1, 2 ..., n-1, n)$, $A\mu_2\nu_2\omega_2/D(1,2, ..., n-1,n)$, ... , $A\mu_{n-1}\nu_{n-1}\omega_{n-1}/D(1,2 ...., n-1, n)$ , $A\mu_n\nu_n\omega_n/D(1,2, ..., n-1,n)$ (die n Markierstoffe $M\mu$ werden jeweils in den Überlappungsbereichen $L\mu\nu\omega$ durch ihre entsprechende Einheit $A\mu\nu\omega$ angeregt und detektiert).

a) Der Aufbau kann ähnlich dem in b) unter I.1) beschriebenen erfolgen. Anstelle eines entsprechenden Karussells für die Photodetektoren $\delta_1\mu$ wird jedoch eine Detektionseinheit D verwendet, welche z.B. (Fall $a_1$) austauschbare Photodetektoren und/oder austauschbare optische Filter (und gegebenenfalls Polarisatoren) $\delta_1\mu$ enthält. Weiter kann D aber auch mehrere Photodetektoren $\delta_1\mu$ enthalten, welchen die jeweilige emittierte Fluoreszenzstrahlung, z.B. (Fall $a_2$) mittels Lichtleitfasern oder Lichtleitfaserbündeln, zugeleitet wird. Entsprechend dem unter b) von I.1) beschriebenen Aufbau erfolgt die Anregung. Die Kombination von Anregung und Detektion der n Markierstoffe $M\mu$ kann nur nacheinander erfolgen.

b) Verwendet man, in Anlehnung an den in c) unter I.1) beschriebenen Aufbau, beispielsweise eine zylinderförmige Probenküvette, so detektiert eine, wie unter a) (Fall $a_1$ oder Fall $a_2$) beschriebene Einheit D die jeweils parallel zur Längsachse der Küvette emittierte Fluoreszenzstrahlung. Radial dazu (und damit jeweils senkrecht zur Längsachse der Küvette) lassen sich wiederum die jeweiligen Einheiten $A\mu\nu\omega$ um die Probenküvette plazieren. Mit einer Einheit D entsprechend Fall $a_1$ kann die Kombination von Anregung und Detektion der n Markierstoffe $M\mu$ nur nacheinander erfolgen. Mit einer Einheit D entsprechend Fall $a_2$ kann die Kombination von Anregung und Detektion der n Markierstoffe $M\mu$ nacheinander und potentiell auch gleichzeitig erfolgen.

I.6) A $(\mu_1\nu_1\omega_1, \mu_2\nu_2\omega_2, ..., \mu_{n-1}\nu_{n-1}\omega_{n-1}, \mu_n\nu_n\omega_n)/D(1,2, ..., n-1, n)$ (die n Markierstoffe $M\mu$ werden jeweils in den Überlappungsbereichen $L\mu\nu\omega$ angeregt und detektiert).
Der Aufbau kann unter Verwendung einer Anregungseinheit A erfolgen, welche z.B. (Fall $a_1$) austauschbare Strahlungsquellen $\alpha_1\mu\nu\omega$ enthält oder Strahlungsquellen $\alpha_1\mu\nu\omega$, deren jeweilige Strahlung, z.B. (Fall $a_2$) mittels Licht-

leitfasern oder Lichtleitfaserbündeln oder kollinearer Überlagerung der einzelnen Strahlen der Strahlungsquellen mittels optischer Elemente, wie z.B. Strahlteiler, dichroitische Strahlteiler, Gitter usw., so geleitet wird, daß sie jeweils am selben Ort der Probenküvette in diese eintritt. Entsprechend kann eine Detektionseinheit D verwendet werden, welche z.B. (Fall $a_1$) austauschbare Photodetektoren und/oder austauschbare optische Filter (und gegebenenfalls Polarisatoren) $\delta_1\mu$ enthält oder auch mehrere Photodetektoren $\delta_1\mu$, welchen die jeweilige emittierte Fluoreszenzstrahlung, z.B. (Fall $a_2$) mittels Lichtleitfasern oder Lichtleitfaserbündeln, zugeleitet wird. Für die Fälle A(Fall $a_1$)/D (Fall $a_1$), A(Fall $a_1$)/D(Fall $a_2$) und A(Fall $a_2$)/D(Fall $a_1$) kann die Kombination von Anregung und Detektion der n Markierstoffe Mμ nur nacheinander erfolgen. Für den Fall A(Fall $a_2$)/D(Fall $a_2$) kann die Kombination von Anregung und Detektion der n Markierstoffe Mμ nacheinander und potentiell auch gleichzeitig erfolgen.
Die geometrische Beziehung der Anregungs- und Detektionseinheiten zueinander entspricht hierbei im wesentlichen den eingangs und in der Schrift WO 94/02570 beschriebenen Verhältnissen.

I.7) $A\mu_1\nu_1\omega_1/A\mu_2\nu_2\omega_2/ \ldots /A\mu_{n-1}\nu_{n-1}\omega_{n-1}/A\mu_n\nu_n\omega_n/D1/D2/ \ldots /Dn-1/Dn$ (die n Markierstoffe Mμ werden gleichzeitig in den Überlappungsbereichen durch die entsprechenden Einheiten Aμ angeregt und gleichzeitig durch die Einheiten Dμ detektiert).
Die gleichzeitige Anregung bzw. Detektion der n Markierstoffe läßt sich im Prinzip mit den geometrischen Verhältnissen durchführen wie sie unter Punkt I.1) in a), Punkt I.2) in b), Punkt I.3) in b), Punkt I.4) in Fall $a_2$ von b), Punkt I.5) in Fall $a_2$ von b) und Punkt I.6) im Fall A(Fall $a_2$)/D(Fall $a_2$) beschrieben wurden.
In Fall II), d.h. die Anregung erfolgt in verschiedenen Probenvolumina, können folgende Verfahren und beispielhafte Aufbaumöglichkeiten der Detektionsgeräte Anwendung finden (n nimmt hierbei z.B. die Werte von 2 bis 10 bzw. 2 bis 6 bzw. 2 bis 4 an):

II.1) A1/D1,A2/D2,...,An-1/Dn-1,An/Dn (die n Markierstoffe Mμ werden jeweils in den Überlappungsbereichen Lμνω durch die entsprechenden Einheiten Aμνω angeregt und durch die jeweiligen Einheiten Dμ detektiert).
Die Anordnung der jeweiligen Paare Aμνω/Dμ entspricht im wesentlichen der eingangs und in Punkt I.1) in a) erläuterten sowie der in der Schrift WO 94/02570 zeichnerisch dargestellten Geometrie. D.h. die optische Achse der Einheit Aμνω (entsprechend der Richtung des Anregungsstrahls) und die optische Achse der entsprechenden Einheit Dμ liegen in einer Ebene, auf welcher die Längsachse der Probenküvette senkrecht steht. Diese beiden optischen Achsen bilden einen Winkel $\chi$, welcher zwischen 0° und 180° liegt, wobei hier Folgendes definiert sein soll: bei Blick von der Einheit Aμνω in deren Strahlrichtung soll dieser Winkel $+\chi$ bzw. $-\chi$ betragen, wenn sich die entsprechende Einheit Dμ rechts bzw. links dieser Blickrichtung befindet. Dies wird durch die Schreibweise Aμνω (+)Dμ bzw. Aμνω(-)Dμ symbolisiert, d.h. $A\mu_1\nu_1\omega_1$(+)D1, $A\mu_2\nu_2\omega_2$(+)D2, $A\mu_3\nu_3\omega_3$(+)D3 usw. bzw. $A\mu_1\nu_1\omega_1$(-)D1, $A\mu_2\nu_2\omega_2$(-)D2, $A\mu_3\nu_3\omega_3$(-)D3 usw. Die Anregung (und Detektion) räumlich verschiedener Probenvolumina erfolgt in der Weise, daß die Anordnung der jeweiligen Paare Aμνω/Dμ in parallelen Ebenen stattfindet. Die Abfolge der Ebenen kann dabei in der Form $A\mu_1\nu_1\omega_1$(+)D1, $A\mu_2\nu_2\omega_2$(+)D2, $A\mu_3\nu_3\omega_3$(+)D3, ..., $A\mu_n\nu_n\omega_n$(+)Dn (äquivalent dazu in der Form $A\mu_1\nu_1\omega_1$(-)D1, $A\mu_2\nu_2\omega_2$(-)D2, $A\mu_3\nu_3\omega_3$(-)D3, ..., $A\mu_n\nu_n\omega_n$(-)Dn) oder auch beispielsweise in der Form $A\mu_1\nu_1\omega_1$(+)D1, $A\mu_2\nu_2\omega_2$(-)D2, $A\mu_3\nu_3\omega_3$(+)D3, ..., $A\mu_{n-1}\nu_{n-1}\omega_{n-1}$(-/+)Dn-1, $A\mu_n\nu_n\omega_n$(+/-)Dn erfolgen. Wenn z.B. die Einheiten Aμνω in einer Reihe angeordnet sind, so sind die Einheiten Dμ in ersterem Fall ebenfalls in einer Reihe auf einer (der "rechten") Seite, in letzerem Fall alternierend in zwei Reihen auf gegenüberliegenden Seiten der Probenküvette angeordnet. Auch ist eine Verschiebung der Ebenen gegeneinander denkbar (Translation). Dies kommt aber üblicherweise nur in Betracht, wenn die von den Einheiten Aμνω jeweils emittierte Anregungsstrahlung nach wie vor senkrecht auf die Aussenseite der Probenküvette auftrifft. Dies ist in der Regel im Falle von Küvetten mit rechtekkigem, nicht aber im Falle von solchen mit rundem Querschnitt gewährleistet.
Weiter können diese Ebenen auch zueinander verdreht sein (Rotation). Bei Blick (Projektion) entlang der Längsache der Küvette bilden somit die zu zwei benachbarten Einheiten Aμνω gehörenden optischen Achsen einen Winkel, der zwischen 0 und 360° liegt. Beispielsweise ergeben sich im Falle von n gleich 2, 3, 4, 5 oder 6 (und bei regelmäßiger, schraubenförmiger Anordnung) entsprechende Winkel zwischen benachbarten Einheiten Aμνω von 180, 120, 90, 72 bzw. 60°. Für n gleich 3, 4, 5 oder 6 implizieren dabei komplementäre Winkel von 240, 270, 288 bzw. 300° (oder -120, -90, -72 bzw. -60°) eine entsprechende gegensinnige Helizität in der Anordnung (im Falle von n gleich 2, d.h. 180° nicht gegeben). Es sei noch angemerkt, daß natürlich auch hier die Ebenen nicht nur entsprechend der Abfolge $A\mu_1\nu_1\omega_1$(+)D1, $A\mu_2\nu_2\omega_2$(+)D2, $A\mu_3\nu_3\omega_3$(+)D3, ,..., $A\mu_n\nu_n\omega_n$(+)Dn (äquivalent dazu in der Abfolge $A\mu_1\nu_1\omega_1$(-)D1, $A\mu_2\nu_2\omega_2$(-)D2, $A\mu_3\nu_3\omega_3$(-)D3, ..., $A\mu_n\nu_n\omega_n$(-)Dn) sondern ebenfalls beispielsweise in der Abfolge $A\mu_1\nu_1\omega_1$(+)D1, $A\mu_2\nu_2\omega_2$(-)D2 , $A\mu_3\nu_3\omega_3$(+)D3, ... , $A\mu_{n-1}\nu_{n-1}\omega_{n-1}$(-/+)Dn-1, $A\mu_n\nu_n\omega_n$(+/-)Dn vorliegen können. Für n gleich 2 (180°) oder 4 (90°) kommt üblicherweise eine Küvette mit rechteckigem Querschnitt zur Anwendung, für n gleich 3 (120°), 5 (72°) oder 6 (60°) wird man üblicherweise (für n gleich 2 oder 4 kann man natürlich auch) eine Küvette mit kreisförmigem Querschnitt verwenden. Die Kombination aus Anregung und Detektion der n Markierstoffe Mμ kann mit den erwähnten Anordnungen sowohl gleichzeitig als auch nacheinander durchgeführt werden.

II.2) $A\mu_1\nu_1\omega_1$/D, $A\mu_1\nu_1\omega_1$/D, ... , $A\mu_{n-1}\nu_{n-1}\omega_{n-1}$/D, $A\mu_n\nu_n\omega_n$/D (die n Markierstoffe $M\mu$ werden jeweils in den Überlappungsbereichen durch die entsprechenden Einheit $A\mu\nu\omega$ angeregt und mit einer Detektionseinheit D, z.B. einem Vielwellenlängendetektor, detektiert.

a) Die Anordnung der Einheiten $A\mu\nu\omega$ kann entsprechend den unter II.1) dargelegten Ausführungen gestaltet werden. Sind die Einheiten $A\mu\nu\omega$ in einer Reihe angeordnet (Fall $a_1$), so kann die Einheit D, bei genügender Größe ihres Strahlungseintrittsfensters oder bei Verwendung einer entsprechenden Abbildungsoptik in der entsprechenden $\chi$-Position ortsfest installiert sein. Andernfalls (Fall $a_2$) muß eine (translatorische) Nachführung in die entsprechende Position erfolgen. Im Falle der übrigen Anordnungen der Einheiten $A\mu\nu\omega$ (Fall $a_3$) muß eine (translatorische und rotatorische) Nachführung der Einheit D in die entsprechenden $\chi$-Positionen erfolgen. Somit kann im Fall $a_1$ die Kombination aus Anregung und Detektion der n Markierstoffe $M\mu$ sowohl gleichzeitig als auch nacheinander, in den Fällen $a_2$ und $a_3$ nur nacheinander erfolgen.

b) Prinzipiell ist auch eine Anordnung in Anlehnung an b) unter Punkt I.3) möglich, d.h. die optische Achse der Einheit D liegt parallel zur Längsachse einer zylinderförmigen Probenküvette, die Einheiten $A\mu\nu\omega$ sind entsprechend den unter II.1) dargelegten Ausführungen angeordnet. Hierbei ergeben sich jedoch unterschiedliche Weglängen, welche die von den jeweiligen Markierstoffen $M\mu$ emittierte Fluoreszenzstrahlung auf dem Weg zur Einheit D durch die Probe zurücklegen muß, bzw. unterschiedliche Raumwinkel, unter welchen die Fluoreszenzstrahlung die Einheit D (bzw. deren Detektionsfenster) trifft. Dies kann zu Ungenauigkeiten in den detektierten Intensitäten führen oder muß entsprechend in den Auswertungen berücksichtigt werden. Prinzipiell kann jedoch in diesen Anordnungen die Kombination aus Anregung und Detektion der n Markierstoffe $M\mu$ sowohl gleichzeitig als auch nacheinander erfolgen.

II.3) $A\mu_1\nu_1\omega_1$/D(1,2,...,n-1,n), $A\mu_2\nu_2\omega_2$/D(1,2,...,n-1,n),..., $A\mu_{n-1}\nu_{n-1}\omega_{n-1}$/D(1,2,...,n-1,n), $A\mu_n\nu_n\omega_n$/D(1,2,...,n-1,n) (die n Markierstoffe $M\mu$ werden jeweils in den Überlappungsbereichen $L\mu\nu\omega$ durch die entsprechenden Einheiten $A\mu\nu\omega$ angeregt und detektiert).

a) Die Anordnungen entsprechen im wesentlichen jenen in a) unter Punkt 11.2) ausgeführten. Anstelle einer Einheit D (z.B. einem Vielwellenlängendetektor) wird jedoch eine Detektionseinheit verwendet, welche z.B. (Fall $a_1$) austauschbare Photodetektoren und/oder austauschbare optische Filter (und gegebenenfalls Polarisatoren) $\delta_1\mu$ enthält. Weiter kann D aber auch mehrere Photodetektoren $\delta_1\mu$ enthalten, welchen die jeweilige emittierte Fluoreszenzstrahlung, z.B. (Fall $a_2$) mittels Lichtleitfasern oder Lichtleitfaserbündeln, zugeleitet wird. Mit einer Einheit D entsprechend Fall $a_1$ kann die Kombination von Anregung und Detektion der n Markierstoffe $M\mu$ nur nacheinander erfolgen, da ein Wechsel der Untereinheiten $\delta_1\mu$ und gegebenenfalls auch eine (translatorische oder translatorische und rotatorische) Nachführung der Einheit D erfolgen muß. Mit einer Einheit D entsprechend Fall $a_2$ kann die Kombination von Anregung und Detektion der n Markierstoffe $M\mu$ nacheinander und potentiell (bei geeigneter Anordnung der Einheiten $A\mu\nu\omega$) auch gleichzeitig durchgeführt werden, wenn, z.B. durch geeignete optische Einrichtungen, eine Bündelung (z.B. durch optische Linse) der von den Markierstoffen $M\mu$ gleichzeitig emittierten Fluoreszenzstrahlung auf die Lichtleitfasern oder -faserbündel erfolgt.

b) Die Anordnungen entsprechen im wesentlichen jenen in b) unter Punkt II.2) ausgeführten. Anstelle einer Einheit D (z.B. einem Vielwellenlängendetektor) wird jedoch wiederum eine Detektionseinheit verwendet, welche z.B. (Fall $a_1$) austauschbare Photodetektoren und/oder austauschbare optische Filter (und gegebenenfalls Polarisatoren) $\delta_1\mu$ enthält. Weiter kann D aber auch mehrere Photodetektoren $\delta_1\mu$ enthalten, welchen die jeweilige emittierte Fluoreszenzstrahlung, z.B. (Fall $a_2$) mittels Lichtleitfasern oder Lichtleitfaserbündeln, zugeleitet wird. Mit einer Einheit D entsprechend Fall $a_1$ kann die Kombination von Anregung und Detektion der n Markierstoffe $M\mu$ nur nacheinander erfolgen. Mit einer Einheit D entsprechend Fall $a_2$ kann die Kombination von Anregung und Detektion der n Markierstoffe $M\mu$ nacheinander und potentiell auch gleichzeitig durchgeführt werden. Die Ausführungen in b) unter Punkt II.2) betreffend die unterschiedlichen Weglängen und Raumwinkel der von den Markierstoffen $M\mu$ emittierten Fluoreszenzstrahlung treffen natürlich auch hier wieder zu.

II.4) $A\mu_1\nu_1\omega_1$/$A\mu_2\nu_2\omega_2$/.../$A\mu_{n-1}\nu_{n-1}\omega_{n-1}$/$A\mu_n\nu_n\omega_n$/D1/D2/.../Dn-1/Dn (die n Markierstoffe werden gleichzeitig in den Überlappungsbereichen $L\mu\nu\omega$ durch die entsprechenden Einheiten $A\mu\nu\omega$ angeregt und gleichzeitig durch die Einheiten $D\mu$ detektiert).

Die gleichzeitige Anregung bzw. Detektion der n Markierstoffe läßt sich im Prinzip mit den Anordnungen und geometrischen Verhältnissen durchführen wie sie in Punkt II.1), Punkt II.2) in Fall $a_1$ von a), Punkt II.2) in b), Punkt II. 3) in Fall $a_2$ von a) und Punkt II.3) in Fall $a_2$ von b) beschrieben wurden.

Die Anordnungen und beispielhaften Aufbaumöglichkeiten von Detektionsgeräten in Fall I, d.h. bei Anregung in denselben Probenvolumina, sind besonders zur zeitlich aufeinander folgenden Anregung der n Markierstoffe $M\mu$ geeignet.

Die Anordnungen und beispielhaften Aufbaumöglichkeiten von Detektionsgeräten in Fall II, d.h. bei Anregung in verschiedenen Probenvolumina, sind besonders zur gleichzeitigen Anregung aller n Markierstoffe $M\mu$ geeignet. Ganz besonders sind darunter solche Anordnungen und beispielhaften Aufbaumöglichkeiten von Detektionsgeräten geeignet, in welchen zudem die jeweils emittierte Fluoreszenzstrahlung an räumlich unterschiedlichen Orten gleichzeitig detektiert werden können.

Generell kann die Anregungsstrahlung der Einheiten $A\mu\nu\omega$ gepulst oder kontinuierlich, d.h. im continouswave-(CW-)Modus, eingestrahlt werden. Weiter kann die Intensität der Anregungsstrahlung jeder Einheit $A\mu\nu\omega$ mit einer Frequenz $f\mu\nu\omega$ moduliert sein, so daß diese Einheit $A\mu\nu\omega$ eine ebenfalls mit $f\mu\nu\omega$ intensitätsmodulierte Fluoreszenzstrahlung des Markierstoffs $M\mu$ anregt, welche von $D\mu$ selektiv gemessen werden kann. Üblicherweise verwendet man dabei Modulationsfrequenzen, welche sich von der Frequenz des Stromnetzes (üblicherweise 50 Hz) sowie den halb- und ganzzahligen Vielfachen dieser Frequenz unterscheiden. Im Falle der gleichzeitigen Anregung und Detektion der von allen Markierstoffen $M\mu$ emittierten Fluoreszenzstrahlung kann über die unterschiedlichen Modulationsfrequenzen $f\mu\nu\omega$ eine Zuordnung der Fluoreszenzstrahlung des jeweiligen Markierstoffes $M\mu$ sowie ein besseres Signal/Rausch-Verhältnis erreicht werden. Zur Detektion der intensitätsmodulierten Fluoreszenzsignale verwendet man üblicherweise das "Lockin-Verfahren".

Eine bevorzugte Ausführungsform des Verfahrens zur Detektion von Markierstoffen in Flussigkeiten, welche markiert worden sind, besteht in der zeitlich nacheinander erfolgenden Anregung der n Markierstoffe $M\mu$ durch ihre entsprechenden Einheiten $A\mu\nu\omega$ in demselben Probenvolumen und der (zeitlich nacheinander erfolgenden) Detektion der jeweils durch $M\mu$ emittierten Fluoreszenzstrahlung.

Eine weitere bevorzugte Ausführungsform des Verfahrens zur Detektion von Markierstoffen in flussigkeiten, welche markiert worden sind, besteht in der gleichzeitig erfolgenden Anregung der n Markierstoffe $M\mu$ durch ihre entsprechenden Einheiten $A\mu\nu\omega$ in demselben Probenvolumen und der gleichzeitig oder zeitlich nacheinander erfolgenden Detektion der jeweils durch $M\mu$ emittierten Fluoreszenzstrahlung mittels eines vielwellenlängendetektors.

Eine weitere bevorzugte Ausführungsform des Verfahrens zur Detektion von Markierstoffen in Flüssigkeiten welche markiert worden sind, besteht in der gleichzeitig erfolgenden Anregung der n Markierstoffe $M\mu$ durch eine polychromatische Einheit A in demselben Probenvolumen und der gleichzeitig oder zeitlich nacheinander erfolgenden Detektion der jeweils durch $M\mu$ emittierten Fluoreszenzstrahlung mittels eines Vielwellenlängendetektors.

Eine weitere bevorzugte Ausführungsform des Verfahrens zur Detektion von Markierstoffen in Flüssigkeiten welche markiert worden sind, besteht in der gleichzeitig erfolgenden Anregung der n Markierstoffe $M\mu$ durch jeweilige, mit der Frequenz $f\mu\nu\omega$ intensitätsmodulierten Einheiten $A\mu\nu\omega$ in demselben Probenvolumen und der gleichzeitig oder zeitlich nacheinander erfolgenden Detektion der jeweiligen, durch $M\mu$ emittierten und intensitätsmodulierten Fluoreszenzstrahlung.

Eine weitere bevorzugte Ausführungsform des Verfahrens zur Detektion von Markierstoffen in Flüssigkeiten welche markiert worden sind, besteht in der gleichzeitig erfolgenden Anregung der n Markierstoffe $M\mu$ durch die Einheiten $A\mu\nu\omega$ in jeweils verschiedenen Probenvolumina und der gleichzeitig oder zeitlich nacheinander erfolgenden Detektion der durch $M\mu$ emittierten Fluoreszenzstrahlung mittels der jeweiligen Einheit $D\mu$.

Bevorzugt verwendet man in den Einheiten $A\mu\nu\omega$ als Strahlungsquellen $\alpha_1\mu\nu\omega$ Halbleiterlaser, Halbleiterdioden oder Festkörperlaser. Ist eine Anregung aller oder eines Teils der Markierstoffe $M\mu$ in deren Hauptabsorptionsbereichen $L\mu(x)$ (mit x gleich z.B. 80, 90, 95 oder 99) erwünscht, so verwendet man in den Einheiten $A\mu\nu\omega$ als Strahlungsquellen $\alpha_1\mu\nu\omega$ bevorzugt Halbleiterlaser, Halbleiterdioden oder Festkörperlaser, welche eine maximale Emission im Spektralbereich von $\lambda_{max}$ - 100 nm bis $\lambda_{max}$ + 20 nm aufweisen, wobei $\lambda_{max}$ die Wellenlänge des Absorptionsmaximums der jeweiligen Markierstoffe $M\mu$ bezeichnet. Diese maximalen Emissionen liegen dann in der Regel innerhalb der entsprechenden Hauptabsorptionsbereiche der jeweiligen Markierstoffe. Alternativ kann aber auch, wie bereits oben erwähnt, eine Anpassung der Hauptabsorptionsbereiche $L\mu(x)$ durch geeignete Wahl von x herbeiführen.

Als Photodetektoren $\delta_1\mu$ in den Einheiten $D\mu$ werden vorteilhaft Halbleiterdetektoren, insbesondere Silicium-Photodioden oder Germanium-Photodioden verwendet.

Ist eine Anregung aller oder eines Teils der Markierstoffe $M\mu$ in deren Hauptabsorptionsbereichen $L\mu(x)$ (mit x gleich z.B. 80, 90, 95 oder 99) erwünscht, so verwendet man als optische Filter in den Photodetektoren $\delta_1\mu$ der Einheiten $D\mu$ bevorzugt Interferenzfilter und/oder Kantenfilter mit einer kurzwelligen Transmissionskante im Bereich von $\lambda_{max}$ bis $\lambda_{max}$ + 80 nm, wobei $\lambda_{max}$ die Wellenlänge des Absorptionsmaximums des jeweils entsprechenden Markierstoffs $M\mu$ bezeichnet.

Gegebenenfalls können auch noch ein oder mehrere NIR-Polarisatoren verwendet werden.

Die folgenden Beispiele sollen die Erfindung näher erläutern.

Beispiel 1:

Eine Mischung aus jeweils 0,5 ppm A ($PcH_2$-(3'-methylphenyloxy)$_4$) und B ($PcH_2$-(3'-methylpiperidino)$_4$) wird in unverbleitem Ottokraftstoff (ROZ 95) gelöst ($PcH_2$ bezeichnet das Phthalocyanin-System, in welchem $Me^1$ (Formel Ia) zweimal Wasserstoff und je ein Rest der Restepaare $R^1$ und $R^4$, $R^5$ und $R^8$, $R^9$ und $R^{12}$ sowie $R^{13}$ und $R^{16}$ in Formel Ia Wasserstoff, der andere Rest 3'-methylphenlyoxy bzw.3'-methylpiperidino entspricht).

Die Absorptionsspektren der Markierstoffe A und B zeigen bei den Wellenlängen von 685 und 754 nm der verwendeten Laser die in Tabelle 1 aufgeführten Extinktionen (in willkürlichen Einheiten):

Tabelle 1

| Laser | Extinktion A | Extinktion B |
|---|---|---|
| 685 nm | 1,3151 | 0,3137 |
| 754 nm | 0,0171 | 0,9954 |

Man erkennt, daß bei Anregung von A mit einem 685-nm-Laser gleichzeitig, jedoch in einem geringeren Ausmaße, auch B angeregt wird.

Bei Anregung von A mit einem 685-nm-Laser und von B mit einem 754-nm-Laser setzt sich das Fluoreszenzsignal von B je aus einem Anteil zusammen, welcher durch Anregung von B mit dem 685-nm-Laser und durch Anregung von B mit dem 754-nm-Laser erhaltenen wird.

Bei Verwendung von A und einem (falschen) Markierstoff B', welcher keinen Überlappungsbereich mit A zeigt, und Anregung von A mit einem 685-nm-Laser und von B' mit einem 754-nm-Laser würde das im Fluoreszenzbereich von B' gemessene Fluoreszenzsignal um den Betrag der nicht erfolgenden Anregung von B' mit dem 685-nm-Laser niedriger ausfallen.

Weiter kann man zur Absicherung der Identität der Markierstoff-Mischung von A und B (bzw. der markierten Flüssigkeit) einerseits die Gesamtintensität der Fluoreszenzstrahlung, welche durch entsprechende, in den Fluoreszenzbereichen der Markierstoffe A und B liegende Filter hindurchtritt, bei gleichzeitiger Anregung, andererseits die Einzelintensitäten bei Anregung mit dem einen und Anregung mit dem anderen Laser bestimmen. Diese drei Messungen bilden zusammen einen (möglichen) unverwechselbaren Fingerabdruck.

Beispiel 2:

Eine Mischung aus jeweils 0,5 ppm B ($PcH_2$-(3'-methylpiperidino)$_4$) und C ($NcH_2$-$(OC_4H_9)_8$) wird in unverbleitem Ottokraftstoff (ROZ 95) gelöst ($NcH_2$ bezeichnet das Naphthalocyanin-System, in welchem $Me^2$ (Formel II) zweimal Wasserstoff entspricht und alle Restepaare $Y^1$ und $Y^2$, $Y^3$ und $Y^4$, $Y^5$ und $Y^6$ sowie $Y^7$ und $Y^8$ in Formel II $OC_4H_9$ entsprechen).

Die Absorptionsspektren der Markierstoffe B und C zeigen bei den Wellenlängen von 754 und 855 nm der verwendeten Laser die in Tabelle 2 aufgeführten Extinktionen (in willkürlichen Einheiten):

Tabelle 2

| Laser | Extinktion B | Extinktion C |
|---|---|---|
| 754 nm | 0,9954 | 0,3824 |
| 855 nm | 0,0037 | 1,9860 |

Man erkennt, daß bei Anregung von B mit einem 754-nm-Laser gleichzeitig, jedoch in einem geringeren Ausmaße, auch C angeregt wird.

Bei Anregung von B mit einem 754-nm-Laser und von C mit einem 855-nm-Laser setzt sich das Fluoreszenzsignal von C je aus einem Anteil zusammen, welcher durch Anregung von C mit dem 754-nm-Laser und durch Anregung von C mit dem 855-nm-Laser erhaltenen wird.

Bei Verwendung von B und einem (falschen) Markierstoff C', welcher keinen Überlappungsbereich mit B zeigt, und Anregung von B mit einem 754-nm-Laser und von C' mit einem 855-nm-Laser würde das im Fluoreszenzbereich von C' gemessene Fluoreszenzsignal um den Betrag der nicht erfolgenden Anregung von C' mit dem 754-nm-Laser niedriger ausfallen.

Weiter kann man zur Absicherung der Identität der Markierstoff-Mischung von B und C, wie bereits in Beispiel 1 ausgeführt, eine Bestimmung der Fluoreszenzsignale bei gleichzeitiger und einzelner Anregung durchführen.

Beispiel 3:

Eine Mischung aus jeweils 0,5 ppm der Verbindungen A, B und C wird in unverbleitem Ottokraftstoff (ROZ 95) gelöst und gleichzeitig mit 3 Lasern bei 685, 754 und 855 nm angeregt. Das resultierende Fluoreszenzlicht wird über drei Bandpaßfilter (durchgelassene Wellenlängen bei 720, 790 und 860 nm) gefiltert und mit Hilfe einer Silicium-Pin-Diode detektiert. Man erhält die in Tabelle 3 aufgeführten Fluoreszenzintensitäten (willkürliche Einheiten) :

Tabelle 3

| Bandpaßfilter | Fluoreszenzsignal |
|---|---|
| 720 nm | 2705 |
| 790 nm | 572 |
| 855 nm | 1589 |

Beispiel 4:

Eine Mischung aus 2/9 (0,22) ppm der Verbindung A, 8/9 (0,88) ppm der Verbindung B und 8/9 (0,88) ppm der Verbindung C wird in unverbleitem Ottokraftstoff (ROZ 95) gelöst und gleichzeitig mit 3 Lasern bei 685, 754 und 855 nm angeregt. Das resultierende Fluoreszenzlicht wird über drei Bandpaßfilter (durchgelassene Wellenlängen bei 720, 790 und 860 nm) gefiltert und mit Hilfe einer Si-Pin-Diode detektiert. Man erhält die in Tabelle 4 aufgeführten Fluoreszenzintensitäten (willkürliche Einheiten):

Tabelle 4

| Bandpaßfilter | Fluoreszenzsignal |
|---|---|
| 720 nm | 2340 |
| 790 nm | 950 |
| 855 nm | 2705 |

Aus den Beispielen 3 und 4 ist ersichtlich, daß (bei entsprechender Kalibrierung) die Unterscheidung unterschiedlicher Verhältnisse der Markierstoffe zueinander sowie unterschiedlicher absoluter Mengen der Markierstoffe durch die Intensitäten ihrer Fluoreszenzsignale möglich ist.

**Patentansprüche**

1. Verfahren zur Detektion von Markierstoffen in Kohlenwasserstoffen und Kohlenwasserstoffgemischen, welche mit mindestens zwei Markierstoffen die im Spektralbereich von 600 bis 1200 nm absorbieren und als Folge davon Fluoreszenzstrahlung emittieren und der Absorptionsbereich mindestens eines Markierstoffes mit dem Absorptionsbereich mindestens eines weiteren Markierstoffes überlappt, markiert Worden sind, **dadurch gekennzeichnet, daß** man Strahlungsquellen verwendet, welche in den Absorptionsbereichen der Markierstoffe Strahlung emittieren, und man die von den Markierstoffen emittierte Fluoreszenzstrahlung nachweist, mindestens eine der Strahlungsquellen Strahlung im überlappenden Absorptionsbereich mindestens eines Markierstoffes mit dem mindestens eines weiteren Markierstoffes emittiert mindestens eine der Strahlungsquellen Strahlung nicht im überlappenden Absorptions bereich mindestens eines Markierstoffes mit dem mindestens eines weiteren Markierstoffes ermittiert und die Anzahl der Strahlungsquellen kleiner oder gleich der Anzahl der Markierstoffe ist.

2. Verfahren nach Anspruch 1, **dadurch gekennzeichnet, daß** man Markierstoffe verwendet, deren jeweilige Wellenlänge des Absorptionsmaximums im Spektralbereich von 600 bis 1200 nm liegt.

3. Verfahren nach den Ansprüchen 1 oder 2, **dadurch gekennzeichnet, daß** man n Markierstoffe zusetzt, wobei n eine ganze Zahl von 2 bis 10 bedeutet.

4. Verfahren nach den Ansprüchen 1 oder 2, **dadurch gekennzeichnet, daß** man n Markierstoffe zusetzt, wobei n eine ganze Zahl von 2 bis 6 bedeutet.

5. Verfahren nach den Ansprüchen 1 oder 2, **dadurch gekennzeichnet, daß** man n Markierstoffe zusetzt, wobei n eine ganze Zahl von 2 bis 4 bedeutet.

6. Verfahren nach den Ansprüchen 1 bis 5, **dadurch gekennzeichnet, daß** man als Markierstoffe Verbindungen zusetzt, die ausgewählt sind aus der Gruppe bestehend aus metallfreien und metallhaltigen Phthalocyaninen, metallfreien und metallhaltigen Naphthalocyaninen, Nickel-Dithiolen-komplexen, Aminiumverbindungen von aromatischen Aminen, Methinfarbstoffen, Quadratsäurefarbstoffen und Krokonsäurefarbstoffen

7. Verfahren nach Anspruch 1, **dadurch gekennzeichnet, daß** man als Strahlungsquellen Halbleiterlaser, Halbleiterdioden oder Festkörperlaser verwendet.

## Claims

1. A method of detecting markers in hydrocarbons and hydrocarbon mixtures marked using at least two markers which absorb in the 600-1200 nm region of the spectrum and reemit fluorescent light and the absorption range of at least one marker overlaps with the absorption range of at least one other marker, which comprises using light sources which emit radiation in the absorption ranges of said markers and detecting the fluorescent light reemited by said markers, at least one of said light sources emitting radiation in the overlapping absorption range of at least one marker with that of at least one other marker, at least one of said light sources emitting radiation that is not in the overlapping absorption range of at least one marker with that of at least one other marker and the number of light sources being less than or equal to the number of markers.

2. The method according to claim 1, wherein markers are used whose respective maximum absorption wavelength is in the 600-1200 nm region of the spectrum.

3. The method according to claim 1 or 2, wherein n markers are added, n being an integer from 2 to 10.

4. The method according to claim 1 or 2, wherein n markers are added, n being an integer from 2 to 6.

5. The method according to claim 1 or 2, wherein n markers are added, n being an integer from 2 to 4.

6. The method according to any of claims 1 to 5, wherein markers added are compounds selected from the group consisting of metal-free and metal-containing phthalocyanines, metal-free and metal- containing naphthalocyanines, nickel-dithiolene complexes, aminium compounds of aromatic amines, methine dyes, squaric acid dyes and croconic acid dyes.

7. The method according to claim 1, wherein said light sources are semiconductor lasers, semiconductor diodes or solid state lasers.

## Revendications

1. Procédé de détection de substances de marquage dans des hydrocarbures et des mélanges d'hydrocarbures, qui ont été marqués par au moins deux substances de marquage, qui absorbent dans le domaine spectral de 600 à 1200 nm et émettent à la suite de cela un rayonnement fluorescent, le domaine d'absorption d'au moins une substance de marquage recouvrant le domaine d'absorption d'au moins une autre substance de marquage, **caractérisé en ce qu'**on utilise des sources de rayonnement qui émettent un rayonnement dans les domaines d'absorption des substances de marquage et on décèle le rayonnement fluorescent émis par les substances de marquage, au moins une des sources de rayonnement émettant un rayonnement dans le domaine d'absorption de recouvrement d'au moins une substance de marquage par ladite au moins une autre substance de marquage, au moins une des sources de rayonnement émettant un rayonnement qui n'est pas dans le domaine d'absorption de recouvrement d'au moins une substance de marquage par ladite au moins une autre substance de marquage, et le nombre de sources de rayonnement étant plus petit ou égal au nombre de substances de marquage.

2. Procédé suivant la revendication 1, **caractérisé en ce qu'**on utilise des substances de marquage dont la longueur d'onde respective du maximum d'absorption se trouve dans le domaine spectral de 600 à 1200 nm.

3. Procédé suivant la revendication 1 ou 2, **caractérisé en ce qu'**on ajoute n substances de marquage, n représentant un nombre entier de 2 à 10.

4. Procédé suivant la revendication 1 ou 2, **caractérisé en ce qu'**on ajoute n substances de marquage, n représentant un nombre entier de 2 à 6.

5. Procédé suivant la revendication 1 ou 2, **caractérisé en ce qu'**on ajoute n substances de marquage, n représentant un nombre entier de 2 à 4.

6. Procédé suivant les revendications 1 à 5, **caractérisé en ce que**, comme substances de marquage, on ajoute des composés qui sont choisis parmi le groupe constitué de phtalocyanines exemptes de métal et contenant du métal, de naphtalocyanines exemptes de métal et contenant du métal, de complexes de nickel-dithiols, de composés aminium d'amines aromatiques, de colorants à groupe méthine, de colorants à acide carré et de colorants à acide croconique.

7. Procédé suivant la revendication 1, **caractérisé en ce que**, comme sources de rayonnement, on utilise un laser à semi-conducteur, des diodes à semi-conducteur ou un laser monolithique.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 9402570 A
- US 5525516 A
- US 5710046 A
- US 5526516 A
- US 5703229 A
- DE 1073739 B
- EP 155780 A
- EP 336213 A
- EP 358080 A
- GB 2168372 A
- GB 2200650 A
- EP 192215 A
- US 3484467 A
- EP 464543 A
- EP 310080 A
- US 4990649 A


### In der Beschreibung aufgeführte Nicht-Patentliteratur


- *Ullmanns Encyklopädie der technischen Chemie,* vol. 7 11, 215-217 435436
- **F.H. MOSER ; A.L. THOMAS.** The Phthalocyanines. CRC Press, 1983
- *J. Am. Chem. Soc.,* 1984, vol. 106, 7404-7410
- **MOSER.** *J.Am. Chem.Soc.*